(19) **Europäisches Patentamt / European Patent Office / Office européen des brevets**

(11) **EP 2 486 920 B1**

(12) **EUROPEAN PATENT SPECIFICATION**

(45) Date of publication and mention
of the grant of the patent:
**26.12.2018 Bulletin 2018/52**

(21) Application number: **09849817.3**

(22) Date of filing: **28.09.2009**

(51) Int Cl.:
*A61K 8/97* (2017.01)    *A61K 31/132* (2006.01)
*A61K 36/48* (2006.01)   *A61P 17/16* (2006.01)
*A61Q 17/00* (2006.01)   *A61Q 19/00* (2006.01)

(86) International application number:
**PCT/JP2009/066744**

(87) International publication number:
**WO 2011/036786 (31.03.2011 Gazette 2011/13)**

(54) **STRESS-ALLEVIATING AGENT COMPRISING PLANT-DERIVED POLYAMINE-CONTAINING EXTRACT AS ACTIVE INGREDIENT**

STRESSLINDERUNGSMITTEL MIT EINEM POLYAMINHALTIGEN PFLANZENEXTRAKT ALS WIRKSTOFF

AGENT DE RELÂCHEMENT DU STRESS COMPRENANT UN EXTRAIT CONTENANT UNE POLYAMINE DÉRIVÉE D'UNE PLANTE COMME INGRÉDIENT ACTIF

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(43) Date of publication of application:
**15.08.2012 Bulletin 2012/33**

(73) Proprietor: **Toyobo Co., Ltd.**
**Osaka-shi**
**Osaka 530-8230 (JP)**

(72) Inventors:
• **KITAZAWA, Hiroaki**
  **Tsuruga-shi**
  **Fukui 914-8550 (JP)**
• **KASUKABE, Yoshihisa**
  **Osaka-shi**
  **Osaka 530-8230 (JP)**
• **KITAGAWA, Masaru**
  **Osaka-shi**
  **Osaka 530-8230 (JP)**

(74) Representative: **Müller-Boré & Partner**
**Patentanwälte PartG mbB**
**Friedenheimer Brücke 21**
**80639 München (DE)**

(56) References cited:
**WO-A1-2008/051080       JP-A- 2001 131 045**
**JP-A- 2004 520 328       JP-A- 2008 156 330**
**JP-A- 2008 239 547       JP-A- 2008 239 548**
**KR-A- 20040 076 092**

• **JIAN-YING WANG GASTROENTEROLOGY vol. 102, 1992, pages 1109 - 1117, XP008160801**
• **, 7 March 2009 (2009-03-07), Retrieved from the Internet: URL:https://www.truthinaging.com/review/department-of-daft-spermine-an-unfortunately-named-antioxidant [retrieved on 2018-05-17]**

EP 2 486 920 B1

**Description**

The present invention relates to use of a substance that enhances a stress-reducing action in various cells and application of such a substance.

Humans and animals are affected by various stresses. Such stresses are strongly related to environmental factors. The environmental factors are probably natural factors such as the ambient temperature (temperature), humidity (dryness), or ultraviolet rays; and factors relating to nutrition, living environment, or lifestyle. Humans and animals are always subjected to stresses due to natural factors and the various resultant ailments or the like are deeply related to a decrease in a cell division rate or degradation of cell functions. For example, skin is constituted by epidermal cells, fibroblasts, and an extracellular matrix such as elastin or collagen for supporting the skin structure outside of epidermal cells and fibroblasts. In young skin, the interaction between the skin tissues is kept constant and, as a result, moisture retention, flexibility, and elasticity are ensured; the skin appears elastic and glossy; and the skin is kept fresh. However, temperature (heat or cold) stress, dryness stress, ultraviolet stress, and the like particularly cause degradation of functions of extracellular matrix or fibroblasts. As a result, the flexibility and moisture retention function of skin are degraded; and the skin loses its elasticity and glossiness and has symptoms such as rough surface, wrinkles, or dullness.

To inhibit or suppress stress at a cellular level, stress-suppressing agents, stress-reducing agents, and stress-preventing agents have been investigated. For example, decomposition products of proteins such as silk protein, keratin protein, and collagen protein (Patent Document 1); essences of original animal and plant sources for Chinese herbal and crude drugs (Patent Document 2); essences of Caprifoliaceous plants (Patent Document 3); nucleic-acid related substances of uridylic acid, uridine, and uracil (Patent Document 4); di-sugar alcohol phosphates of $C_3$ to $C_6$ sugar alcohols and/or derivatives of such compounds (Patent Document 5); and the like are known. However, such substances do not provide sufficient action or efficacy. Thus, there are no anti-stress agents or stress-reducing agents that provide sufficient action and efficacy.

Polyamine is a generic name for aliphatic hydrocarbons having two or more primary amino groups, which are natural substances universally present in living things. The physiological actions of polyamine have been attracting attention. It is known that polyamine mainly has the following physiological actions: (1) stabilizing and changing the structure of nucleic acid due to the interaction between polyamine and nucleic acid; (2) action of promoting various nucleic-acid synthetic systems; (3) activating protein synthetic systems; (4) stabilizing cell membranes and enhancing membrane permeability of substances; (5) eliminating active oxygen; and (6) promoting cell proliferation. However, it is not known at all whether polyamine provides an anti-stress action or a stress-reducing action, in particular, on skin cells (fibroblasts).

Patent Document 1: Japanese Unexamined Patent Application Publication No. 2003-81868
Patent Document 2: Japanese Unexamined Patent Application Publication No. 2001-187725
Patent Document 3: Japanese Unexamined Patent Application Publication No. 2001-213795
Patent Document 4: Japanese Unexamined Patent Application Publication No. 2005-330213
Patent Document 5: Japanese Unexamined Patent Application Publication (Translation of PCT Application) No. 2005-506676

Fig. 1 illustrates the cell vitalization action of soybean extracts prepared in an Example, on normal human skin fibroblasts under a low-temperature stress condition.
Fig. 2 illustrates the cell vitalization action of soybean extracts prepared in an Example, on normal human skin fibroblasts under a high-temperature stress condition.
Fig. 3 illustrates the cell vitalization action of wheat extracts prepared in an Example, on normal human skin fibroblasts under a low-temperature stress condition.
Fig. 4 illustrates the cell vitalization action of wheat extracts prepared in an Example, on normal human skin fibroblasts under a high-temperature stress condition.
Fig. 5 illustrates comparison among the cell vitalization actions of synthetic polyamines, soybean extracts prepared in an Example, and wheat extracts prepared in an Example, on normal human skin fibroblasts under a low-temperature stress condition.
Fig. 6 illustrates comparison among the cell vitalization actions of synthetic polyamines, soybean extracts prepared in an Example, and wheat extracts prepared in an Example, on normal human skin fibroblasts under a high-temperature stress condition.

An object of providing an anti-stress action or a stress-reducing action on mammals, in particular, humans, is considerably important. Several anti-stress agents and stress-reducing agents that are derived from animals and plants have been found. However, these agents do not actually provide sufficient or stable efficacy for industrial applications. Thus, novel anti-stress agents and stress-reducing agents are being investigated.

An object of the present invention is to provide, for the purpose of preventing or suppressing stress at a cellular level, use of a cosmetic composition containing a plant-derived polyamine-containing extract that overcomes the above-described problems and the cosmetic composition has a temperature stress-reducing action, a temperature stress-suppressing action and a temperature stress-preventing action.

The inventors of the present invention have performed thorough studies on how to achieve the objects. As a result, the inventors have found that polyamine-containing plant extracts sufficiently provide a temperature stress-reducing action, a temperature stress-suppressing action and a temperature stress-preventing action on skin cells (in particular, fibroblasts). Thus, the present invention has been accomplished.

Specifically, the present invention encompasses the following inventions (1) to (5).

(1) Use of a cosmetic composition for reducing, suppressing or preventing temperature-stress in skin cells of a mammal, wherein said cosmetic composition comprises, as an active ingredient, a plant-derived polyamine-containing extract, and wherein the temperature stress is due to heat and cold.

(2) The use according to (1), wherein the plant-derived polyamine-containing extract is an extract derived from at least one material selected from the group consisting of a soybean seed, a soybean germ, a soybean embryo, a soybean bud, a wheat seed, a wheat germ, a wheat embryo, a wheat bud, soymilk, and soybean curd refuse.

(3) The use according to (1) or (2), wherein the polyamine includes an aliphatic hydrocarbon compound having two or more primary amino groups.

(4) The use according to any one of (1) to (3), wherein the polyamine is at least one compound selected from the group consisting of 1,3-diaminopropane, putrescine, cadaverine, cardine, spermidine, homospermidine, aminopropylcadaverine, termine, spermine, thermospermine, canavalmine, aminopentylnorspermidine, N,N-bis(aminopropyl)cadaverine, homospermine, caldopentamine, homocaldopentamine, caldohexamine, and homocaldohexamine.

(5) The use according to any one of (1) to (4), wherein the polyamine is at least one compound selected from the group consisting of putrescine, spermidine, and spermine.

The stress-reducing agent used in the present invention is expected to provide an excellent temperature stress-reducing action, temperature stress-suppressing action and temperature stress-preventing action for cells and contains a plant-derived polyamine-containing extract serving as an active component. Use of cosmetics that contain such a stress-reducing agent can be provided.

In the present invention, "stress-reducing action" indicates that degradation of cell functions caused in animal cells under various stresses is reduced and, specifically indicates that, for example, degradation of a cell division function, degradation of a cell activation function, degradation of a cell vitalization function, or degradation of a cell immunity function, each of which is caused in animal or human cells under various stresses, is reduced. "Anti-stress action and stress-reducing action" indicates that, for example, in skin cells, degradation of functions of skin cells due to accumulation of structural changes of a basement membrane caused by stress is reduced to thereby, for example, prevent and ameliorate wrinkles, slack, hardening, or the like in skin and maintain an elastic, young, and healthy skin condition; and indicates that, for example, in hair papilla cells or hair matrix cells, degradation of functions of hair papilla cells or hair matrix cells due to stress is reduced to thereby, for example, maintain the hair cycle and suppress loss of hair. "Stress-reducing action" also indicates an anti-stress action, a stress-suppressing action, a stress-preventing action, a stress-inhibiting action, or a stress protective action.

In the present invention, "stress-reducing agent" means an agent having a stress-reducing action and also indicates an anti-stress agent, a stress-suppressing agent, a stress-preventing agent, a stress-inhibiting agent, or a stress protective agent.

In the present invention, "stress" indicates a stress due to high temperature (heat) and low temperature (cold).

In the present invention, "plant extract" indicates a substance obtained by extraction from a plant and/or a processed plant.

In the present invention, an agent having a stress-reducing action is also an anti-stress agent, a stress-suppressing agent, a stress-preventing agent, a stress-inhibiting agent, or a stress protective agent.

In the present invention, "plant-derived polyamine-containing extract" indicates a (natural) polyamine-containing plant extract obtained from a plant and/or a processed plant. In the present invention, "plant-derived polyamine-containing extract" is sometimes referred to as "plant extract containing polyamine and/or plant extract containing polyamine as an active component". In the present invention, "plant extract" indicates a substance obtained by extraction from a plant and/or a processed plant. The concentration of polyamine contained in a plant-derived polyamine-containing extract is, in M (mole/liter), generally 0.000001 to 5 M, preferably 0.000005 to 3 M, and more preferably 0.00001 to 2 M; and in wt%, generally 0.0001 to 95 wt%, preferably 0.001 to 75 wt%, and more preferably 0.005 to 50 wt%.

"Plant-derived polyamine-containing extract" may be a low-purity product that is not purified for increasing the polyamine concentration or a high-purity product that has been purified with an ion-exchange resin or the like and has a considerably high polyamine concentration. A low-purity product of soybean/wheat germ extract powder has a polyamine concentration of about 0.1 to 0.2%. A high-purity product of soybean/wheat germ extract powder having been purified with an ion-exchange resin can have a high polyamine concentration of about 30 to 50 wt%. When such an extract is in the form of an aqueous solution, the concentration is 1/10 to 1/100 of the above-described concentrations.

"Polyamine" in the present invention is a generic name for aliphatic hydrocarbons having two or more primary amino groups, which are natural substances universally present in living things. Twenty or more polyamines have been found: for example, 1,3-diaminopropane, putrescine, cadaverine, cardine, spermidine, homospermidine, aminopropylcadaverine, termine, spermine, thermospermine, canavalmine, aminopentylnorspermidine, N,N-bis(aminopropyl)cadaverine, homospermine, caldopentamine, homocaldopentamine, caldohexamine, and homocaldohexamine. Representative polyamines are putrescine, spermidine, and spermine.

"Putrescine" is one of representative polyamines, a general natural substance universally present in living things, and an aliphatic hydrocarbon compound having two primary amino groups. "Spermidine" is one of representative polyamines, a general natural substance universally present in living things, and an aliphatic hydrocarbon compound having three primary amino groups. "Spermine" is one of representative polyamines, a general natural substance universally present in living things, and an aliphatic hydrocarbon compound having four primary amino groups.

Non-limiting examples of a plant from which a plant extract containing polyamine and/or a plant extract containing polyamine as an active component is obtained include: dicotyledons, monocotyledons, herbaceous plants, arbor plants, Cucurbitaceous plants, Solanaceous plants, Gramineous plants, Brassicaceous plants, Leguminous plants, Malvaceous plants, Compositous plants, Chenopodiaceous plants, plant extracts of the foregoing, and plant essences of the foregoing. Specific examples include: sweet potato, tomato, cucumber, squash, melon, watermelon, tobacco, *Arabidopsis thaliana,* pimento, eggplant, bean, aroid, spinach, carrot, strawberry, potato, rice, corn, alfalfa, wheat, barley, soybean, rape, sorghum, eucalyptus, aspen, kenaf, *Eucommia ulmoides,* sugarcane, sugar beet, cassava, sago palm, chenopod, lily, orchid, carnation, rose, chrysanthemum, petunia, torenia, antirrhinum, cyclamen, gypsophila, geranium, sunflower, lawn grass, cotton, velvet shank, *Lyophyllum shimeji, Tricholoma matsutake,* shiitake mushroom, mushrooms, ginseng, *Agaricus,* turmeric, Asian ginseng, citrus, green tea, black tea, oolong tea, banana, kiwi, fermented soybeans (natto), soymilk, soybean essence, wheat essence, germ essence, embryo essence, fruit juice, soybean curd refuse (okara), rice germ, wheat germ, barley germ, soybean germ, corn germ, milo germ, and sunflower germ.

Preferred plants are monocotyledons and dicotyledons and, more preferably, Gramineous plants and Leguminous plants. Particularly preferred plants and processed plants are corn, mushrooms, soybean, wheat, fermented soybeans (natto), soymilk, soybean curd refuse (okara), wheat germs, soybean germs, corn germs, soybean essence, wheat essence, germ essence, and embryo essence; and, more preferably, soybean seeds, soybean germs, soybean embryos, soybean buds, wheat seeds, wheat germs, wheat embryos, wheat buds, soymilk, and soybean curd refuse (okara).

A plant tissue from which a plant extract containing polyamine and/or a plant extract containing polyamine as an active component is obtained is not particularly limited. Such a plant tissue is preferably in the form of a seed or in a growing period. In the case of a plant in a growing period, a plant extract used in the present invention can be obtained from the entirety or a partial tissue of the plant. Non-limiting examples of such an extraction portion include the whole plant, a flower, a flower bud, an ovary, a fruit, a leaf, a cotyledon, a stem, a bud, a root, a seed, a dry seed, an embryo, and a germ; preferably, a fruit, a leaf, a stem, a bud, a seed, a dry seed, a germ, and an embryo; and particularly preferably, a seed, a dry seed, a germ, and an embryo.

A plant from which a plant extract containing polyamine and/or a plant extract containing polyamine as an active component is obtained may be a processed plant. Such a processed plant is processed by a procedure in which an extract is collected from the plant with water, an organic solvent, a mixture of water and an organic solvent, or the like, by an impregnation method, a distillation method, a compression method, an ultrasonic method, a supercritical-fluid method, a subcritical-fluid method, or the like, under a condition of a low temperature, room temperature, or a high temperature. Furthermore, for example, a processed product obtained by subjecting a plant or an extract collected from a plant to a process such as fermentation may be used: for example, a plant essence, soymilk, soybean curd refuse (okara), flour, a fermented essence, fermented soybeans (natto), or the like.

A method by which a plant extract containing polyamine and/or a plant extract containing polyamine as an active component is obtained may be a method in which the extraction is performed by adding an acid solution to a plant and/or a processed plant such that an acidic condition is achieved. "Acidic condition" denotes a condition in which the pH is 6 or less. By achieving a pH satisfying the acidic condition in the extraction, a polyamine composition can be efficiently and stably collected from a plant tissue. This advantage can be uniformly achieved in a pH region of 6 or less; however, the pH is preferably 4 or less and, particularly preferably, 2 or less. The lower limit of the pH corresponds to the pH of an undiluted solution of the acid solution used and is not particularly limited; however, the lower limit of the pH is preferably 0 to 2.

Compared with a plant extract collected with an organic solvent such as ethanol or methanol, by obtaining a plant extract under an acidic condition (with an acid aqueous solution), the polyamine-amount collection percentage is high; in particular, compound polyamine, which is sparingly soluble in water, can be made to be dissolved in water under the acidic condition; and the content of free polyamine, which is extracted with an organic solvent at a low efficiency, is also increased. Polyamine exhibits an excellent stability under the acidic condition and the stability of polyamine and other active components in a plant extract is enhanced. In addition, by obtaining a plant extract under the acidic condition (with an acid aqueous solution), natural active components other than polyamine are also collected. Examples of the natural active components include saccharides such as monosaccharides and oligosaccharides; peptides; and proteins. The presence of polyamine and natural active components other than polyamine further enhances an anti-stress action and a stress-reducing action. In addition, the collected plant extract is in the form of an aqueous solution and hence the merit of high safety is expected compared with an organic solvent.

The acid solution added for achieving the acidic condition may be a mineral acid such as hydrochloric acid, sulfuric acid, or phosphoric acid; an organic acid such as acetic acid, propionic acid, butyric acid, oxalic acid, malonic acid, succinic acid, fumaric acid, maleic acid, malic acid, lactic acid, tartaric acid, citric acid, or benzoic acid; or acid water. Such an acid solution is preferably, for example, an inorganic acid or an organic acid such as 0.01 N to 6 N hydrochloric acid, sulfuric acid, nitric acid, acetic acid, phosphoric acid, trichloroacetic acid, sulfosalicylic acid, formic acid, citric acid, lactic acid, or 0.1 to 10% perchloric acid; and particularly preferably, 0.0625 to 1 N hydrochloric acid, 0.25 to 5% perchloric acid, 0.1 to 1 N sulfuric acid, or the like.

In the extraction step, a polyphenol adsorbent may be added. The addition of a polyphenol adsorbent can facilitate collection of a plant extract containing polyamine and/or a plant extract containing polyamine as an active component. The polyphenol adsorbent is not particularly limited as long as the polyphenol adsorbent can adsorb polyphenols. However, the polyphenol adsorbent is preferably polyvinyl polypyrrolidone (PVPP), polyvinyl pyrrolidone (PVP), polyethylene glycol (PEG), or the like; and, particularly preferably, polyvinyl polypyrrolidone (PVPP). Such a polyphenol adsorbent may be a commercially available product: for example, POLYCLAR (registered trademark) (manufactured by ISP Inc.), Divergan (registered trademark) (manufactured by BASF), Dowex (registered trademark) -1, PVP-40, or the like. By adding a polyphenol adsorbent after the addition of an acid solution to a plant and/or a processed plant, an increase in a collection amount, a yield, or a purity of a plant extract containing polyamine and/or a plant extract containing polyamine as an active component is expected. Furthermore, the addition of a polyphenol adsorbent can suppress an entry of polyphenols and the like into a plant extract, the polyphenols and the like negatively affecting, in cells and skin cells, vitalization, anti-oxidation, anti-aging, collagen production, hyaluronic acid production, or a stress-reducing action. The amount of such a polyphenol adsorbent added is preferably 0.1 to 30% (w/v), more preferably 0.5 to 20% (w/v), and still more preferably 1 to 10% (w/v).

The collection amount of a plant extract containing polyamine and/or a plant extract containing polyamine as an active component can be increased by adding an acid solution to a plant and/or a processed plant such that the acidic condition is achieved, if necessary, adding a polyphenol adsorbent, then crushing, grinding, and mixing the plant and/or the processed plant. In particular, in the case of a plant tissue, which has cell walls, the cell walls are desirably damaged. In the case of a processed plant or a plant essence, which does not have cell walls, crushing or grinding for damaging cell walls is not necessary. Crushing or grinding may be performed by, for example, a method in which a mixer, a blender, a homogenizer, a mortar, an ultrasonic crusher, or the like is employed.

Polyamine contained in a plant and/or a processed plant is sufficiently extracted to an acid solution (liquid fraction) and the liquid fraction is then subjected to separation from residue or precipitation, such as centrifugal separation or filtration separation. The collected liquid fraction contains a large amount of polyamine and "plant extract containing polyamine and/or plant extract containing polyamine as an active component" is obtained. If necessary, a polyamine-containing plant extract may be subjected to a deionization treatment or a purification treatment by an ion-exchange method, a membrane fractionation method, a gel filtration method, or an electrodialysis method. By performing at least one of these methods, a plant extract having a higher purity in terms of polyamine can be obtained. In the case of the ion-exchange method, a plant-extract solution is passed through a column filled with an ion-exchange resin to thereby isolate polyamine serving as an active component from impurities such as amino acids, peptides, proteins, and saccharides. In the ion-exchange resin used, the ion-exchange group may be a sulfonic group, a sulfopropyl group, a phosphate group, a carboxylmethyl group, an aminoethyl group, a diethylamino group, a quaternary aminoethyl group, a quaternary ammonium group, or the like. A cation-exchange resin or an anion-exchange resin may be used. When a cation-exchange resin is used, the cation-exchange resin adsorbs polyamine serving as an active component. Accordingly, non-adsorption substances are sufficiently separated and then the active component is eluted with an acidic solution such as sulfuric acid or hydrochloric acid or a solution of a salt such as sodium chloride. When an anion-exchange resin is used, the anion-exchange resin does not adsorb polyamine serving as an active component and hence a non-adsorption fraction containing the active component is collected. In the case of the membrane fractionation method, a plant extract is subjected to ultrafiltration (UF) through a UF membrane that is a cellulose-based membrane, a cellulose acetate-based membrane, a polysulfone-based membrane, a polyamide-based membrane, a polyacrylonitrile-based membrane, a polytetrafluoroethylene-based membrane, a polyester-based membrane, a polypropylene-based membrane, or the like, and that has a fractionation molecular-weight range of 1,000 to 100,000, to thereby collect a filtrate containing polyamine serving as an active component. In addition, the active-component solution is deionized by being subjected to nanofiltration (NF) through a NF membrane having a salt-blocking percentage of 30 to 80%. In the case of the gel filtration method, a plant extract is neutralized and passed through a column filled with gel-filtration carriers to be fractionated in terms of molecular weight. Thus, polyamine serving as an active component is collected. Such gel-filtration carriers are dextran-based carriers, acrylamide-based carriers, agarose-based carriers, cellulose-based carriers, polyvinyl-based carriers, glass-based carriers, polystyrene-based carriers, or the like and have a fractionation molecular weight range of 100 to 100,000. In the case of the electrodialysis method, electrodialysis is performed while a plant extract and a saline solution are alternately supplied into sections between cation-exchange membranes and anion-exchange membranes. Conditions for electrodialysis include an initial current density of 0.5 to 15 A/dm$^2$ and a voltage of 0.1 to 1.5 V/vessel.

Polyamine, a plant extract containing polyamine and/or a plant extract containing polyamine as an active component, which may be used itself as a stress-reducing agent, is used in the form of a mixture as cosmetics.

The plant-derived polyamine-containing extract should be added, to a cosmetic composition, in an amount with which the action of the plant-derived polyamine-containing extract is provided. In general, the amount is preferably 0.001 to 50 mass%, more preferably 0.01 to 30 mass%, still more preferably 0.1 to 10 mass%, and particularly preferably 0.5 to 5 mass%.

The mixing concentration (M: mole/liter) of polyamine is determined in accordance with, for example, the degree of absorption, the degree of action, the form of a product, or frequency in use and is not particularly limited: however, the mixing concentration is generally 0.00001 to 100 mM, preferably 0.00005 to 75 mM, and more preferably 0.0001 to 50 mM. In the case of skin external preparations directly applied to human skin such as liquid preparations, cosmetics (e.g., cream, emulsion, lotion, face lotion, or ointment), or pharmaceuticals, the concentration is generally 0.01 to 10,000 μM, preferably 0.05 to 1,000 μM, more preferably 0.1 to 100 μM, and particularly preferably 1 to 10 μM. In the case of solid preparations such as oral preparations, a single dose is generally 0.01 to 10,000 μM, preferably 0.05 to 1,000 μM, more preferably 0.1 to 100 μM, and particularly preferably 1 to 10 μM.

"Plant-derived polyamine-containing extract" used in the present invention is derived from a natural plant and hence is safer than chemically synthesized products. In addition, a plant-derived polyamine-containing extract is prepared from a plant, which is convenient. Furthermore, a plant-derived polyamine-containing extract contains at least three polyamines of putrescine, spermidine, and spermine and hence the necessity of individually preparing these polyamines is eliminated. In the case of a plant-derived polyamine-containing extract, plant materials that are cultivated or commercially available should be used; in particular, wheat seeds and soybean seeds are sold at low prices among plant materials and can also be secured in large amounts and the production cost of such a polyamine-containing extract is lower than that of chemically synthesized products. As for cosmetics, natural substances, in particular, plant-derived substances are demanded. In addition, a plant-derived extract contains natural components other than polyamine and the action of each natural component or the interaction between the natural components may enhance the action of polyamine. Furthermore, natural components other than polyamine (for example, saccharides such as monosaccharides and oligosaccharides, peptides, and proteins) are expected to act as active components (stress-reducing action). Therefore, a stress-reducing agent containing a plant-derived polyamine-containing extract serving as an active component used in the present invention is highly advantageous.

Since a plant-derived polyamine-containing extract used in the present invention contains natural components other than polyamine in the extract, the extract has less odor peculiar to polyamine and higher stability than polyamine. Thus, the extract can be applied to cosmetics, quasi drugs, medical supplies, sanitary goods, pharmaceuticals, foods, or drinks, without degrading the quality.

In a stress-reducing agent used in the present invention, if necessary, polyamine serving as an essential component may be mixed with a component or an additive that is used for cosmetics, quasi drugs, foods, drinks, pharmaceuticals, or the like as long as advantages of the present invention are not degraded.

Examples of oils and fats include avocado oil, almond oil, fennel oil, perilla oil, olive oil, orange oil, orange roughy oil, sesame oil, cocoa butter, camomile oil, carrot oil, cucumber oil, beef tallow, beef tallow acid, candlenut oil, safflower oil, soybean oil, camellia oil, corn oil, rape oil, persic oil, castor oil, cottonseed oil, peanut oil, turtle oil, mink oil, yolk oil, cocoa butter, palm oil, palm kernel oil, Japan wax, coconut oil, beef tallow, lard, hydrogenated oil, and hydrogenated castor oil.

Examples of waxes include beeswax, carnauba wax, spermaceti wax, lanolin, liquid lanolin, reduced lanolin, hard lanolin, candelilla wax, montan wax, and shellac wax.

Examples of mineral oils include liquid paraffin, Vaseline, paraffin, ozokerite, ceresin, microcrystalline wax, polyethylene powder, squalene, squalane, and pristane.

Examples of fatty acids include natural fatty acids such as lauric acid, myristic acid, palmitic acid, stearic acid, behenic acid, oleic acid, 12-hydroxystearic acid, undecylenic acid, tall oil, and lanolin fatty acid; and synthetic fatty acids such as isononanoic acid, caproic acid, 2-ethylbutanoic acid, isopentanoic acid, 2-methylpentanoic acid, 2-ethylhexanoic acid, and isopentanoic acid.

Examples of alcohols include natural alcohols such as ethanol, isopropanol, lauryl alcohol, cetanol, stearyl alcohol, oleyl alcohol, lanolin alcohol, cholesterol, and phytosterol; synthetic alcohols such as 2-hexyldecanol, isostearyl alcohol, and 2-octyldodecanol; and polyhydric alcohols such as ethylene oxide, ethylene glycol, diethylene glycol, triethylene glycol, ethylene glycol monoethyl ether, ethylene glycol monobutyl ether, diethylene glycol monomethyl ether, diethylene glycol monoethyl ether, polyethylene glycol, propylene oxide, propylene glycol, polypropylene glycol, 1,3-butylene glycol, glycerin, batyl alcohol, pentaerythritol, sorbitol, mannitol, glucose, and sucrose.

Examples of esters include isopropyl myristate, isopropyl palmitate, butyl stearate, hexyl laurate, myristyl myristate, oleyl oleate, decyl oleate, octyldodecyl myristate, hexyldecyl dimethyloctanoate, cetyl lactate, myristyl lactate, diethyl phtalate, dibutyl phtalate, lanolin acetate, ethylene glycol monostearate, propylene glycol monostearate, and propylene glycol dioleate.

Examples of metal soap include aluminum stearate, magnesium stearate, zinc stearate, calcium stearate, zinc palmitate, magnesium myristate, zinc laurate, and zinc undecylenate.

Examples of gummy matter and water-soluble polymer compounds include gum arabic, gum benzoin, gum dammar, guaiac resin, Irish moss, karaya gum, gum tragacanth, gum carob, quince seeds, agar, casein, dextrin, gelatin, pectin, starch, carrageenan, carboxyalkyl chitin, chitosan, hydroxyalkyl chitin, low-molecular-weight chitosan, chitosan salts, sulfated chitin, phosphorylated chitin, alginic acid, alginates, hyaluronic acid, hyaluronates, chondroitin sulfuric acid, heparin, ethyl cellulose, methyl cellulose, carboxymethyl cellulose, carboxyethyl cellulose, sodium carboxyethyl cellulose, hydroxyethyl cellulose, hydroxypropyl cellulose, nitrocellulose, crystalline cellulose, polyvinyl alcohol, polyvinyl methyl ether, polyvinyl pyrrolidone, polyvinyl methacrylate, polyacrylate, polyalkylene oxides such as polyethylene oxide and polypropylene oxide, crosslinked polymers of polyalkylene oxides, carboxyvinyl polymers, and polyethyleneimine.

Examples of surfactants include anionic surfactants (carboxylates, sulfonates, sulfates, and phosphates), cationic surfactants (amine salts and quaternary ammonium salts), amphoteric surfactants (carboxylic-acid amphoteric surfactants, sulfate amphoteric surfactants, sulfonic-acid amphoteric surfactants, and phosphate amphoteric surfactants), nonionic surfactants (ether nonionic surfactants, ether-ester nonionic surfactants, ester nonionic surfactants, block-polymer nonionic surfactants, and nitrogen-containing nonionic surfactants), and other surfactants (natural surfactants, derivatives of protein hydrolysates, polymeric surfactants, titanium-silicon-containing surfactants, and fluorocarbon surfactants.

Examples of vitamins include: in the vitamin A group, retinol, retinal (vitamin A1), dehydroretinal (vitamin A2), carotin, and lycopene (provitamin A); in the vitamin B group, thiamine hydrochloride, thiamine sulfate (vitamin B1), riboflavin (vitamin B2), pyridoxine (vitamin B6), cyanocobalamin (vitamin B12), folic acids, nicotinic acids, pantothenic acids, biotins, choline, and inositols; in the vitamin C group, ascorbic acid and derivatives of ascorbic acid; in the vitamin D group, ergocalciferol (vitamin D2), cholecalciferol (vitamin D3), and dihydrotachysterol; in the vitamin E group, tocopherol, derivatives of tocopherol, and ubiquinones; and, in the vitamin K group, phytonadione (vitamin K1), menaquinone (vitamin K2), menadione (vitamin K3), and menadiol (vitamin K4).

Examples of amino acids include: valine, leucine, isoleucine, threonine, methionine, phenylalanine, tryptophan, lysine, glycine, alanine, asparagine, glutamine, serine, cysteine, cystine, tyrosine, proline, hydroxyproline, aspartic acid, glutamic acid, hydroxylysine, arginine, ornithine, and histidine; and amino acid derivatives such as sulfates, phosphates, nitrates, citrates of the foregoing and pyrrolidone carboxylate.

Examples of skin-lightening agents include: ascorbic acid, ascorbic acid derivatives, sulfur, placenta hydrolysate, ellagic acid, ellagic acid derivatives, kojic acid, kojic acid derivatives, glucosamine, glucosamine derivatives, arbutin, arbutin derivatives, hydroxycinnamic acid, hydroxycinnamic acid derivatives, glutathione, arnica essence, *Scutellaria* root essence, mulberry bark essence, *Bupleurum* root essence, saposhnikovia root essence, *Ganoderma* mycelium culture, *Ganoderma* mycelium culture extract, basswood essence, peach leaf essence, rose fruit essence, sophora root essence, *Sanguisorba officinalis* essence, Japanese angelica root essence, coix seed essence, oriental persimmon leaf essence, rhubarb essence, moutan bark essence, hamamelis essence, marronnier essence, *Hypericum erectum* essence, and oil-soluble *Glycyrrhiza* essence.

Examples of moisture retention agents include hyaluronic acid, polyglutamic acid, serine, glycine, threonine, alanine, collagen, hydrolyzed collagen, hydronectin, fibronectin, keratin, elastin, royal jelly, heparin chondroitin sulfate, glycerophospholipid, glyceroglycolipid, sphingophospholipid, sphingoglycolipid, linoleic acid, linoleates, eicosapentaenoic acid, eicosapentaenoates, pectin, *Lactobacillus bifidus* fermented products, lactic acid fermented products, yeast extract, *Ganoderma lucidum* mycelium culture, *Ganoderma lucidum* mycelium culture extract, wheat germ oil, avocado oil, rice germ oil, jojoba oil, soybean phospholipid, γ-oryzanol, marsh mallow essence, coix seed essence, rehmannia root essence, jujube essence, squill essence, *Aloe arborescens* essence, burdock essence, rosemary essence, arnica essence, and wheat bran.

Examples of hair-growth agents include glyceride pentadecanoate, *Coleus* essence, gentian essence, pine cone essence, royal jelly essence, *Sasa veitchii* essence, t-flavanone, 6-benzylaminopurine, *Swertia japonica* essence, carpronium chloride, minoxidil, finasteride, adenosine, nicotinamide, mulberry root essence, rehmannia root essence, and 5-aminolevulinic acid.

Examples of extracts and essences from animals, plants, and crude drugs include those from *Uncaria gambir* (gambir), *Angelica keiskei*, acerola, althea, arnica, avocado, *Hydrangea macrophylla* Seringe var. *thunbergii* Makino (sweet hydrangea leaf), aloe, aloe vera, nettle, *Ginkgo biloba* (ginkgo leaf and ginkgo nut), fennel, turmeric, *Asarum sieboldii* (Asiasarum root), Japanese apricot, *Quercus salicina, Arctostaphylos uva-ursi, Rosa multiflora* (rose fruit), *Isodonis Japonicus, Astragalus* root, *Scutellaria baicalensis* (*Scutellaria* root), *Prunus sargentii* (cherry bark), *Phellodendron amurense* (*Phellodendron* bark), *Coptis japonica* (*Coptis* rhizome), *Panax ginseng* (ginseng), *Hypericum erectum, Lamium album, Nasturtium officinale,* orange, *Polygala tenuifolia* (*Polygala* root), Prunella *vulgaris, Polygonum multiflorum* (*Polygonum* root), *Sophora* (*Sophora* flower), *Artemisia Princeps* (mugwort leaf), *Curcuma zedoaria* (zedoary), *Pueraria lobata* (*Pueraria* root), *Valerian* (*Valerian* root), *Anthemis Nobilis, Trichosanthes kirilowii* (*Trichosanthes* root), *Artemisia capillaris* (*Artemisia capillaris* flower), *Glycyrrhiza, Tussilago farfara* (coltsfoot flower and coltsfoot leaf), *Rubus idaeus,* kiwi fruit, *Platycodon* (*Platycodon* root), chrysanthemum (chrysanthemum flower), *Catalpa ovata* (*Catalpa* fruit), citrus fruit (immature orange), *Citrus tachibana* (*Citrus tachibana* peel), cucumber, *Aralia cordata* and *Notopterygium* (*Notopterygium* rhizome and *Aralia* rhizome), *Prunus armeniaca* (apricot kernel), *Lycium chinense* (*Lycium* root bark, *Lycium* fruit, and *Lycium* leaf), *Sophora flavescens* (*Sophora* root), *Cinnamomum camphora, Sasa veitchii, Citrus paradisi* fruit, *Cinnamomum zeylanicum* (cinnamon bark), *Schizonepeta tenuifolia* (*Schizonepeta* spike), *Cassia obtusifolia* (*Cassia* seed), *Ipomoea purpurea* and *Ipomoea nil* (pharbitis seed), *Carthamus tinctorius* (safflower), gallnut, comfrey, copaiba, gardenia (gardenia fruit), gentian, *Magnolia obovata* (*Magnolia* bark), *Achyranthes bidentata* (*Achyranthes* root), Evodia (Evodia fruit), burdock, *Schizandra chinensis* (*Schizandra* fruit), rice, rice bran, wheat, *Bupleurum scorzonerifolium* (*Bupleurum* root), saffron, *Saponaria officinalis, Crataegus cuneata* (*Crataegus* fruit), *Zanthoxylum piperitum* (*Zanthoxylum* fruit), salvia, *Panax notoginseng* (*Panax notoginseng* root), *Lentinus edodes* (shiitake mushroom), *Rehmannia glutinosa* (*Rehmannia* root), *Quisqualis indica* (*Quisqualis indica* seed), *Lithospermum erythrorhizon* (*Lithospermum* root), *Perilla ocymoides* (perilla leaf and perilla seed), Oriental persimmon (persimmon calyx), *Paeonia albiflora* (peony root), *Plantago asiatica* (*Plantago* seed and *Plantago* herb), *Zingiber officinale* (ginger), *Acorus calamus* (calamus), *Ligustrum lucidum* (*Ligustrum lucidum* fruit), *Spiraea ulmaria,* Betula *alba, Lonicera japonica* (*Lonicera* flower and *Lonicera* leaf and stem), English ivy, yarrow, European elder, adzuki (adzuki bean), elder (*Sambucus sieboldiana*), common mallow, *Cnidium officinale* (*Cnidium* rhizome), *Swertia japonica* (*Swertia* herb), mulberry (mulberry root bark and mulberry leaf), jujube, soybean, *Aralia elata, Panax japonicus* (*Panax japonicus* rhizome), *Anemarrhena asphodeloides* (*Anemarrhena* rhizome), *Sanguisorba officinalis, Houttuynia cordata* (*Houttuynia* herb), *Cordyceps sinensis* (plant worm), pepper, *Physalis alkekengi* (*Physalis alkekengi* root), thyme, green tea, black tea, clove, *Citrus unshiu* (*Citrus unshiu* peel), camellia, *Centella asiatica,* capsicum, *Angelica acutiloba* (Japanese angelica root), *Calendula officinalis, Citrus aurantium* (bitter orange peel), *Sanguisorba officinalis,* corn (corn silk), *Eucommia ulmoides* (*Eucommia* bark and *Eucommia* leaf), tomato, *Nandina domestica* (*Nandina domestica* fruit), garlic, barley (malt), *Dictamnus* (*Dictamnus* root bark), *Ophiopogon japonicus* (*Ophiopogon* tuber), parsley, batata, *Mentha arvensis* (*Mentha* herb), hamamelis, rose, *Eriobotrya japonica* leaf (loquat leaf), *Poria cocos* (*Poria* sclerotium), grape, grape leaf, dishcloth gourd, linden, moutan (moutan bark), hop, *Rosa rugosa* (*Rosa rugosa* flower), pine needle, marronnier, rosemary, soapberry, melissa, melilot, *Chaenomeles speciosa,* bean sprout, peach (peach kernel and peach leaf), *Belamcanda chinensis,* betel palm (betel nut), *Leonurus* (*Leonurus* herb), cornflower, strawberry geranium, bayberry (myrica), *Alnus firma, Coix lacryma-jobi* var. *ma-yuen* (coix seed), *Artemisia mongolia, Artemisia montata,* lavender, apple fruit, *Ganoderma lucidum* (Leyss. ex. Fr.) Karst, lemon fruit, *Forsythia* (forsythia fruit), *Astragalus,* Geranium herb, *Scopolia* (*Scopolia* rhizome), comb of cock, bovine/human placenta extract, extract/decomposed extract of swine/bovine stomach/duodenum/intestine, water-soluble collagen, water-soluble collagen derivatives, hydrolyzed collagen, elastin, hydrolyzed elastin, water-soluble elastin derivatives, silk protein, decomposition products of silk protein, and decomposition products of bovine blood-cell protein.

Examples of microorganism culture metabolites include yeast essence, zinc-containing yeast essence, germanium-containing yeast essence, selenium-containing yeast essence, magnesium-containing yeast essence, fermented rice essence, *Euglena* extract, and lactic fermentation products of non-fat dry milk.

Examples of α-hydroxy acids include glycolic acid, citric acid, malic acid, tartaric acid, and lactic acid.

Examples of inorganic pigments include silicic acid anhydride, magnesium silicate, talc, kaoline, bentonite, mica, titanium mica, bismuth oxychloride, zirconium oxide, magnesium oxide, zinc oxide, titanium oxide, calcium carbonate, magnesium carbonate, iron oxide yellow, iron red, iron oxide black, ultramarine blue, chromium oxide, chromium hydroxide, carbon black, and calamine.

Examples of ultraviolet absorbing agents include p-aminobenzoic acid derivatives, salicylic acid derivatives, anthranilic acid derivatives, coumarin derivatives, aminoacid compounds, benzotriazole derivatives, tetrazole derivatives, imidazoline derivatives, pyrimidine derivatives, dioxane derivatives, camphor derivatives, furan derivatives, pyrrone derivatives, nucleic acid derivatives, allantoin derivatives, nicotinic acid derivatives, vitamin B6 derivatives, oxybenzone, benzophenone, guaiazulene, shikonin, baicalin, baicalein, and berberine.

Examples of astringents include lactic acid, tartaric acid, succinic acid, citric acid, allantoin, zinc chloride, zinc sulfate, zinc oxide, calamine, zinc p-phenolsulfonate, potassium aluminum sulfate, resorcin, ferric chloride, and tannic acid.

Examples of antioxidants include ascorbic acid, ascorbates, stearates, tocopherol, tocopherol ester derivatives, nordihydroguaiaretic acid, butylated hydroxytoluene (BHT), butylated hydroxyanisole (BHA), p-hydroxyanisole, propyl gallate, sesamol, sesamolin, and gossypol.

Examples of anti-inflammatory agents include ichthammol, indomethacin, kaoline, salicylic acid, sodium salicylate, methyl salicylate, acetylsalicylic acid, diphenhydramine hydrochloride, d/dl-camphor, hydrocortisone, guaiazulene, chamazulene, chlorpheniramine maleate, glycyrrhizinic acid, glycyrrhizinates, glycyrrhetinic acid, and glycyrrhetinates.

Examples of sterilizing agents include acrinol, sulfur, benzalkonium chloride, benzethonium chloride, methylrosaniline chloride, cresol, calcium gluconate, chlorhexidine gluconate, sulfamine, Mercurochrome, lactoferrin, and hydrolyzed lactoferrin.

Examples of hair-care agents include selenium disulfide, alkyl isoquinolinium bromide solutions, zinc pyrithione, biphenamine, thianthol, castoreum tincture, ginger tincture, capsicum tincture, quinine hydrochloride, strong aqueous ammonia, potassium bromate, sodium bromate, and thioglycolic acid.

Examples of fragrance materials include natural animal fragrance materials such as musk, civet, castoreum, and ambergris; plant fragrance materials such as anisie essential oil, angelica essential oil, ilang essential oil, orris essential oil, fennel essential oil, orange essential oil, cananga essential oil, caraway essential oil, cardamom essential oil, guaiacum wood essential oil, cumin essential oil, Lindera essential oil, cassia essential oil, cinnamon essential oil, geranium essential oil, copaiba balsam essential oil, coriander essential oil, perilla essential oil, cedarwood essential oil, citronella essential oil, jasmine essential oil, ginger-grass essential oil, cedar essential oil, spearmint essential oil, peppermint essential oil, star-anise essential oil, tuberose essential oil, clove essential oil, neroli essential oil, gaultheria essential oil, Tolu balsam essential oil, patchouli essential oil, rose essential oil, palmarosa essential oil, cypress essential oil, thuja essential oil, sandalwood essential oil, petitgrain essential oil, bay essential oil, vetiver essential oil, bergamot essential oil, Peru balsam essential oil, bois de rose essential oil, camphor tree essential oil, mandarin essential oil, eucalyptus essential oil, lime essential oil, lavender essential oil, linaloe essential oil, lemongrass essential oil, lemon essential oil, rosemary essential oil, and Japanese mint essential oil; and other synthetic fragrance materials.

Examples of pigments/coloring agents include red cabbage pigment, red-kerneled rice pigment, madder pigment, annatto pigment, sepia pigment, turmeric pigment, sophora pigment, krill pigment, persimmon pigment, caramel, gold, silver, gardenia pigment, corn pigment, onion pigment, tamarind pigment, spirulina pigment, buckwheat entire-plant pigment, cherry pigment, laver pigment, hibiscus pigment, grape juice pigment, marigold pigment, purple sweet potato pigment, purple yam pigment, lac pigment, and rutin.

Examples of sweeteners include sugar, sweet hydrangea leaf, fructose, arabinose, galactose, xylose, mannose, maltose, honey, glucose, miraculin, and monellin.

Examples of nutritional additives include calcined shell calcium, cyanocolabamin, yeast, wheat germ, soybean germ, yolk powder, hemicellulose, and hemoferrum.

In addition, there are hormones, metal-ion blocking agents, pH adjusting agents, chelating agents, antiseptic/antifungal agents, refrigerants, stabilizing agents, emulsifying agents, animal/plant proteins, decomposed animal/plant proteins, animal/plant polysaccharides, decomposed animal/plant polysaccharides, animal/plant glycoproteins, decomposed animal/plant glycoproteins, blood-flow increasing agents, anti-inflammatory agents, antiallergic agents, cell vitalization agents, keratolytic agents, wound-healing agents, foaming agents, thickening agents, oral-care agents, deodorants, bittering agents, condiments, enzymes, and the like.

An agent used in the present invention may be used in any desired dosage form and may be blended to provide skin/hair care cosmetics; in the form of ampules, capsules, powder, granules, pills, tablets, solid, liquid, gel, bubble, emulsion, cream, ointment, sheets, mousse, or the like.

Specific examples of cosmetics include internal/external pharmaceutical preparations; basic cosmetics, facial cleansing agents, and skin cleansing agents such as face lotion, emulsion, cream, ointment, lotion, oil, and facial pack; hair cosmetics such as shampoo, rinse, hair treatment, hair cream, pomade, hair spray, hair dressing, permanent solution, hair tonic, hairdye, and hair-growth agent; makeup cosmetics such as foundation, white powder, face powder, lipstick, blush, eye shadow, eye liner, mascara, eyebrow pencil, and eyelash; finishing cosmetics such as manicure agents; perfumes; bath agents; dentifrices; oral refrigerants/gargles; liquid-odor-suppressing agents/deodorants; sanitary goods; sanitary cottons; and wet tissue papers.

A stress-reducing agent used in the present invention is suitably applied to humans. However, as long as such a stress-reducing agent is expected to provide its action and advantage, the stress-reducing agent may also be applied to animals other than humans.

EXAMPLES

The present invention will be described in further detail with the following examples. However, the present invention is not restricted to the examples.

(Polyamine analysis method)

The content of polyamine in a sample can be examined by the following method. Polyamines in plants are free polyamines, compound polyamines, and bound polyamines. Although these polyamines are extracted by different methods, all these polyamines can be analyzed (Plant Cell Physiol., 43(2), 196-206, 2002, J. Nutr. Biochem., 4, 66-70, 1993, Biosci. Biotech. Biochem., 61(9), 1582-1584, 1997). A method of analyzing free polyamines in plant seeds will be described in detail as a specific example. A diluted internal standard solution (1,6-hexanediamine or 1,7-diaminoheptane; internal standard amount = 7.5 or 12 nmol) and a 5% aqueous solution of perchloric acid (5-20 mL relative to 1.0 g of the fresh weight of a sample) are added to about 0.1 to 1.0 g of a soybean seed. This sample is sufficiently ground to perform extraction with a Polytron mixer at room temperature. The solution provided by the grinding is subjected to centrifugal separation at 4°C and at 35,000 $\times$ g for 20 minutes and the supernatant, which serves as a free-polyamine solution, is collected. A microtube with a screw cap is filled with 100 to 400 $\mu$L of the free-polyamine solution (plant extract, purified plant extract), 200 $\mu$L of a saturated aqueous solution of sodium carbonate, and 200 $\mu$L of a dansyl chloride/acetone solution (10 mg/mL) and these solutions are lightly mixed. The tube is tightly closed with the cap, then covered with an aluminum foil, and heated in a water bath at 60°C for an hour to thereby perform dansylation. After the tube is left to cool, 200 $\mu$L of an aqueous solution of proline (100 mg/mL) is added to the tube and mixed. The tube is covered with an aluminum foil and heated again in the water bath for 30 minutes. The solution is left to cool, then subjected to nitrogen-gas flow to thereby remove acetone, subsequently mixed with 600 $\mu$L of toluene, and strongly mixed. The tube is left to stand to provide two separate phases. The upper toluene layer is collected into a 300 $\mu$L microtube. A nitrogen gas is blown to the collected toluene to completely remove toluene. To the tube, 200 $\mu$L of methanol is added so that the dansylated free polyamines are dissolved. The amounts of the free polyamines of putrescine, spermidine, and spermine are determined by analysis according to an internal standard method with a high-performance liquid chromatography system connected to a fluorescence detector (excitation wavelength: 365 nm, emission wavelength: 510 nm). The HPLC columns are $\mu$Bondapak C18 (manufactured by Waters Corporation, 027324, 3.9 $\times$ 300 mm, particle size: 10 $\mu$m). The content of the polyamines in the sample is calculated by determining the area of the peaks of the polyamines and the internal standard from the HPLC chart of the standard solution and the sample.

(EXAMPLE 1A: preparation of soybean-derived polyamine-containing extract)

A diluted internal standard solution (1,7-diaminoheptane; internal standard amount = 1200 nmol) and 500 mL of a 5% aqueous solution of perchloric acid were added to 100 g of soybean germs (manufactured by FOR YOU corporation) and left at room temperature for an hour. Then, the resultant solution was mixed with 16 g of Polyclar VT (manufactured by ISP Inc.) serving as a polyphenol adsorbent. The soybean germs were sufficiently crushed with a blender mixer and then subjected to extraction under the acidic condition by being left at room temperature for 30 minutes. The crushed substance was subjected to centrifugal separation at 2°C and at 22,000 $\times$ g for 20 minutes and the liquid fraction was collected. The liquid fraction was neutralized with a 30% solution of sodium hydroxide. The resultant solution was defined as a plant extract (soybean-germ extract; LGS). The plant extract contained 57.1 mg of polyamines in total: 23.5 mg of putrescine, 24.0 mg of spermidine, and 9.6 mg of spermine. The collected plant extract was passed through a column filled with a cation-exchange resin (AG 50W-X4, 200-400 mesh, H+ type, manufactured by Bio-Rad Laboratories, Inc.) to make the resin adsorb the polyamines. The column was washed by sequentially passing a 0.7 N NaCl/0.1 M sodium phosphate solution (pH 8.0), water, and 1 N hydrochloric acid. After impurities were removed, the polyamines were eluted with 6 N hydrochloric acid and neutralized with 30% sodium hydroxide. The resultant solution was defined as a purified plant extract (purified soybean-germ extract; HGS). The purified plant extract contained 50.4 mg of polyamines in total: 20.4 mg of putrescine, 22.3 mg of spermidine, and 7.7 mg of spermine. The plant extract (soybean-germ extract; LGS) and the purified plant extract (purified soybean-germ extract; HGS) were deionized with an electrodialyser (ACILYZER, manufactured by ASTOM Corporation), concentrated by freeze drying, and used in various evaluations.

(EXAMPLE 1B: preparation of soybean-derived polyamine-containing extract)

Soybean-germ samples (each 1 kg, soybean-germ powder manufactured by FOR YOU corporation) were mixed with 0.1 N, 0.5 N, and 1 N hydrochloric acid solutions (each 6 L). Then, each resultant solution was mixed with 80 g of Divergan F (manufactured by BASF) serving as a polyphenol adsorbent and subjected to extraction under the acidic condition by being stirred with a Three-one Motor at room temperature for 2 hours. The stirred substance was subjected to centrifugal separation at 4°C and at 12,000 $\times$ g for 30 minutes and the liquid fraction was collected. The liquid fraction was neutralized with a 30% solution of sodium hydroxide. The resultant solution was then subjected to centrifugal separation at 4°C and at 12,000 × g for 30 minutes and the liquid fraction was collected. This solution was defined as a plant extract (soybean-germ extract; LGS).

The plant extract in terms of 0.1 N hydrochloric acid contained 416.1 mg of polyamines in total: 217.9 mg of putrescine, 163.5 mg of spermidine, and 34.7 mg of spermine. The plant extract in terms of 0.5 N hydrochloric acid contained 433.0 mg of polyamines in total: 226.5 mg of putrescine, 170.0 mg of spermidine, and 36.5 mg of spermine. The plant extract in terms of 1 N hydrochloric acid contained 446.76 mg of polyamines in total: 232.4 mg of putrescine, 174.4 mg of spermidine, and 39.96 mg of spermine. The plant extracts (soybean-germ extracts; LGS) were deionized with an electrodialyser (ACILYZER, manufactured by ASTOM Corporation), concentrated by freeze drying, and used in various evaluations.

From the results, it has been confirmed that a plant extract containing polyamine and/or a plant extract containing polyamine as an active component can be collected with the hydrochloric-acid concentrations of 0.1 N, 0.5 N, and 1 N. Thus, the preparation method excellent in terms of safety and handling has been found.

(EXAMPLE 1C: preparation of soybean-derived polyamine-containing extract)

Soybean-germ samples (each 1 kg, soybean-germ powder manufactured by FOR YOU corporation) were mixed with 0.1 N, 0.5 N, and 1 N sulfuric acid solutions (each 6 L). Then, each resultant solution was mixed with 80 g of Divergan F (manufactured by BASF) serving as a polyphenol adsorbent and subjected to extraction under the acidic condition by being stirred with a Three-one Motor at room temperature for 2 hours. The stirred substance was subjected to centrifugal separation at 4°C and at 12,000 × g for 30 minutes and the liquid fraction was collected. The liquid fraction was neutralized with a 30% solution of sodium hydroxide. The resultant solution was then subjected to centrifugal separation at 4°C and at 12,000 × g for 30 minutes and the liquid fraction was collected. This solution was defined as a plant extract (soybean-germ extract; LGS).

The plant extract in terms of 0.1 N sulfuric acid contained 421.6 mg of polyamines in total: 220.8 mg of putrescine, 165.7 mg of spermidine, and 35.1 mg of spermine. The plant extract in terms of 0.5 N sulfuric acid contained 427.1 mg of polyamines in total: 223.7 mg of putrescine, 167.9 mg of spermidine, and 35.5 mg of spermine. The plant extract in terms of 1 N sulfuric acid contained 438.1 mg of polyamines in total: 229.4 mg of putrescine, 172.2 mg of spermidine, and 36.5 mg of spermine. The plant extracts (soybean-germ extracts; LGS) were deionized with an electrodialyser (ACILYZER, manufactured by ASTOM Corporation), concentrated by freeze drying, and used in various evaluations.

From the results, it has been confirmed that a plant extract containing polyamine and/or a plant extract containing polyamine as an active component can be collected with the sulfuric-acid concentrations of 0.1 N, 0.5 N, and 1 N. Thus, the preparation method excellent in terms of safety and handling has been found.

(EXAMPLE 2A: preparation of wheat-derived polyamine-containing extract)

A diluted internal standard solution (1,7-diaminoheptane; internal standard amount = 1200 nmol) and 500 mL of a 5% aqueous solution of perchloric acid were added to 100 g of wheat germs (roast type, manufactured by Nisshin Pharma Inc.) and left at room temperature for an hour. Then, the resultant solution was mixed with 16 g of Polyclar VT (manufactured by ISP Inc.) serving as a polyphenol adsorbent. The wheat germs were sufficiently crushed with a blender mixer and then subjected to extraction under the acidic condition by being left at room temperature for 30 minutes. The crushed substance was subjected to centrifugal separation at 2°C and at 22,000 × g for 20 minutes and the liquid fraction was collected. The liquid fraction was neutralized with a 30% solution of sodium hydroxide. The resultant solution was defined as a plant extract (wheat-germ extract; LGW). The plant extract contained 44.6 mg of polyamines in total: 6.4 mg of putrescine, 23.9 mg of spermidine, and 14.3 mg of spermine. The collected plant extract was further passed through a column filled with a cation-exchange resin (AG 50W-X4, 200-400 mesh, H+ type, manufactured by Bio-Rad Laboratories, Inc.) to make the resin adsorb the polyamines. The column was washed by sequentially passing a 0.7 N NaCl/0.1 M sodium phosphate solution (pH 8.0), water, and 1 N hydrochloric acid. After impurities were removed, the polyamines were eluted with 6 N hydrochloric acid and neutralized with 30% sodium hydroxide. The resultant solution was defined as a purified plant extract (purified wheat-germ extract; HGW). The purified plant extract contained 40.1 mg of polyamines in total: 5.7 mg of putrescine, 21.5 mg of spermidine, and 12.9 mg of spermine. The plant extract (wheat-germ extract; LGW) and the purified plant extract (purified wheat-germ extract; HGW) were deionized with an electrodialyser (ACILYZER, manufactured by ASTOM Corporation), concentrated by freeze drying, and used in various evaluations.

(EXAMPLE 2B: preparation of wheat-derived polyamine-containing extract)

Wheat-germ samples (each 1 kg, particularly defatted wheat-germ fine powder (20 kg) manufactured by Nisshin Pharma Inc.) were mixed with 0.1 N, 0.5 N, and 1 N hydrochloric acid solutions (each 4 L). Then, each resultant solution was mixed with 80 g of Divergan F (manufactured by BASF) serving as a polyphenol adsorbent and subjected to extraction under the acidic condition by being stirred with a Three-one Motor at room temperature for 2 hours. The stirred substance was subjected to centrifugal separation at 4°C and at 12,000 $\times$ g for 30 minutes and the liquid fraction was collected. The liquid fraction was neutralized with a 30% solution of sodium hydroxide. The resultant solution was then subjected to centrifugal separation at 4°C and at 12,000 $\times$ g for 30 minutes and the liquid fraction was collected. This solution was defined as a plant extract (wheat-germ extract; LGW). The plant extract in terms of 0.1 N hydrochloric acid contained 296 mg of polyamines in total: 48.4 mg of putrescine, 201.6 mg of spermidine, and 46 mg of spermine. The plant extract in terms of 0.5 N hydrochloric acid contained 282.8 mg of polyamines in total: 48.4 mg of putrescine, 183.6 mg of spermidine, and 50.8 mg of spermine. The plant extract in terms of 1 N hydrochloric acid contained 312.2 mg of polyamines in total: 51.2 mg of putrescine, 204.4 mg of spermidine, and 56.6 mg of spermine. The plant extracts (wheat-germ extracts; LGW) were deionized with an electrodialyser (ACILYZER, manufactured by ASTOM Corporation), concentrated by freeze drying, and used in various evaluations.

From the results, it has been confirmed that a plant extract containing polyamine and/or a plant extract containing polyamine as an active component can be collected with the hydrochloric-acid concentrations of 0.1 N, 0.5 N, and 1 N. Thus, the preparation method excellent in terms of safety and handling has been found.

(EXAMPLE 2C: preparation of wheat-derived polyamine-containing extract)

Wheat-germ samples (each 1 kg, particularly defatted wheat-germ fine powder (20 kg) manufactured by Nisshin Pharma Inc.) were mixed with 0.1 N, 0.5 N, and 1 N sulfuric acid solutions (each 4 L). Then, each resultant solution was mixed with 80 g of Divergan F (manufactured by BASF) serving as a polyphenol adsorbent and subjected to extraction under the acidic condition by being stirred with a Three-one Motor at room temperature for 2 hours. The stirred substance was subjected to centrifugal separation at 4°C and at 12,000 $\times$ g for 30 minutes and the liquid fraction was collected. The liquid fraction was neutralized with a 30% solution of sodium hydroxide. The resultant solution was then subjected to centrifugal separation at 4°C and at 12,000 $\times$ g for 30 minutes and the liquid fraction was collected. This solution was defined as a plant extract (wheat-germ extract; LGW). The plant extract in terms of 0.1 N sulfuric acid contained 292 mg of polyamines in total: 50 mg of putrescine, 195.6 mg of spermidine, and 46.4 mg of spermine. The plant extract in terms of 0.5 N sulfuric acid contained 308.2 mg of polyamines in total: 50 mg of putrescine, 201.6 mg of spermidine, and 56.6 mg of spermine. The plant extract in terms of 1 N sulfuric acid contained 308.4 mg of polyamines in total: 52.8 mg of putrescine, 197.2 mg of spermidine, and 58.4 mg of spermine. The plant extracts (wheat-germ extracts; LGW) were deionized with an electrodialyser (ACILYZER, manufactured by ASTOM Corporation), concentrated by freeze drying, and used in various evaluations.

From the results, it has been confirmed that a plant extract containing polyamine and/or a plant extract containing polyamine as an active component can be collected with the sulfuric-acid concentrations of 0.1 N, 0.5 N, and 1 N. Thus, the preparation method excellent in terms of safety and handling has been found.

(EXAMPLE 3A: evaluation of stress-reducing (anti-stress) action of soybean-germ-derived polyamine-containing plant extract (low-temperature stress))

The plant extract (soybean-germ extract; LGS) described in EXAMPLE 1B was used as a plant extract containing soybean-germ-derived polyamine and/or a plant extract containing soybean-germ-derived polyamine as an active component. The evaluation was performed by the following procedure. Fibroblasts (106-05, manufactured by Cell Applications, Inc.) were cultured in a T-75 flask. When the fibroblasts reached 70 to 80% confluency, the culture medium was aspirated. The fibroblasts were washed with 10 ml of PBS twice. The cells were released by the addition of 500 $\mu$l of trypsin/PBS and the reaction was terminated by the addition of 500 $\mu$l of a 10% FCS-containing DMEM (11995-073, manufactured by GIBCO). The cells were mixed with 10 ml of PBS and collected into a 50 ml tube. The cells were mixed with another 10 ml of PBS and collected. The cells were collected at 1,000 rpm for 5 minutes and the supernatant was aspirated. The resultant solution was dissolved in 3 ml of a 1% FCS-containing DMEM and the number of cells was counted. The solution was adjusted to be $4 \times 10^4$ cells/ml ($1 \times 10^4$ cells/250 $\mu$l) with a 1% FCS-containing DMEM and 250 $\mu$l of the resultant solution was each injected into wells of 48-well plates. The cells were incubated overnight in a $CO_2$ incubator set at 37°C. Then, samples (2.5 $\mu$l per well) were added to wells and the cells were incubated for 3 days in a $CO_2$ incubator set at 37°C (under no-stress condition) and set at 32°C (low-temperature stress condition). The experiment was performed with N = 4. After the culture medium was removed with an aspirator, the wells were washed twice with PBS. After 200 $\mu$l of a 5% FCS-containing DMEM (21063-029, manufactured by GIBCO) was added to each well, 20 $\mu$l of a viable-cell count reagent SF (07553-54, manufactured by NACALAI TESQUE, INC.) was added and the reaction was allowed to occur in the $CO_2$ incubator for 5 hours. Abs 490/630 was measured and a cell vitalization percentage was calculated with the following formula.

$$\text{Cell vitalization percentage} = (As - Ab)/(Ac - Ab) \times 100$$

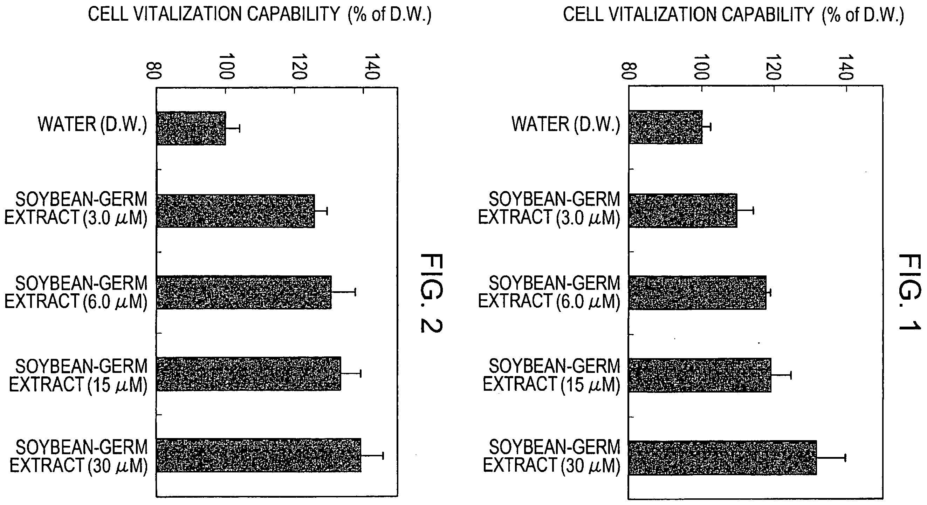

Herein, As represents the Abs 490/630 of a sample; Ac represents the Abs 490/630 of the control (water); and Ab represents the Abs 490/630 of a well to which the cells were not seeded but the 5% FCS-containing DMEM and the viable-cell count reagent SF were added. The evaluation results are illustrated in Fig. 1 in relative values with respect to the cell vitalization action in the control (water added), this cell vitalization action being defined as 100. The concentrations illustrated are the concentrations of polyamine.

The cell vitalization capability in the water-added (control) under the low-temperature stress condition at 32°C was 75% lower than that under the no-stress condition at 37°C (room temperature). As is clear from Fig. 1, in all the culture media to which the soybean-germ extract was added, significantly (significant level: 1% or 5%) high cell vitalization capabilities were observed compared with the water-added control. Thus, it has been confirmed that the samples provide an excellent stress-reducing (anti-stress) action. Alternatively, when the plant extracts (soybean-germ extracts; LGS) described in EXAMPLES 1A and 1C were used, results similar to that in the case where the plant extract described in EXAMPLE 1B was used were also obtained. In summary, by applying polyamines to skin, degradation of fibroblasts due to low-temperature stress is reduced (suppressed) and hence improvement of the feel of skin can be expected.

(EXAMPLE 3B: evaluation of stress-reducing (anti-stress) action of soybean-germ-derived polyamine-containing plant extract (high-temperature stress))

The plant extract (soybean-germ extract; LGS) described in EXAMPLE 1B was used as a plant extract containing soybean-germ-derived polyamine and/or a plant extract containing soybean-germ-derived polyamine as an active component. The evaluation was performed by the following procedure. Fibroblasts (106-05, manufactured by Cell Applications, Inc.) were cultured in a T-75 flask. When the fibroblasts reached 70 to 80% confluency, the culture medium was aspirated. The fibroblasts were washed with 10 ml of PBS twice. The cells were released by the addition of 500 $\mu$l of trypsin/PBS and the reaction was terminated by the addition of 500 $\mu$l of a 10% FCS-containing DMEM (11995-073, manufactured by GIBCO). The cells were mixed with 10 ml of PBS and collected into a 50 ml tube. The cells were mixed with another 10 ml of PBS and collected. The cells were collected at 1,000 rpm for 5 minutes and the supernatant was aspirated. The resultant solution was dissolved in 3 ml of a 1% FCS-containing DMEM and the number of cells was counted. The solution was adjusted to be $4 \times 10^4$ cells/ml ($1 \times 10^4$ cells/250 $\mu$l) with a 1% FCS-containing DMEM and 250 $\mu$l of the resultant solution was each injected into wells of 48-well plates. The cells were incubated overnight in a $CO_2$ incubator set at 37°C. Then, samples (2.5 $\mu$l per well) were added to wells and the cells were incubated for 3 days in a $CO_2$ incubator set at 37°C (under no-stress condition) and set at 42°C (high-temperature stress condition). The experiment was performed with N = 4. After the culture medium was removed with an aspirator, the wells were washed twice with PBS. After 200 $\mu$l of a 5% FCS-containing DMEM (21063-029, manufactured by GIBCO) was added to each well, 20 $\mu$l of a viable-cell count reagent SF (07553-54, manufactured by NACALAI TESQUE, INC.) was added and the reaction was allowed to occur in the $CO_2$ incubator for 5 hours. Abs 490/630 was measured and a cell vitalization percentage was calculated with the following formula.

$$\text{Cell vitalization percentage} = (As - Ab)/(Ac - Ab) \times 100$$

Herein, As represents the Abs 490/630 of a sample; Ac represents the Abs 490/630 of the control (water); and Ab represents the Abs 490/630 of a well to which the cells were not seeded but the 5% FCS-containing DMEM and the viable-cell count reagent SF were added. The evaluation results are illustrated in Fig. 2 in relative values with respect to the cell vitalization action in the control (water added), this cell vitalization action being defined as 100. The concentrations illustrated are the concentrations of polyamine.

The cell vitalization capability of the water-added (control) under the high-temperature stress condition at 42°C was 70% lower than that under the no-stress condition at 37°C (room temperature). As is clear from Fig. 2, in all the culture media to which the soybean-germ extract was added, significantly (significant level: 1% or 5%) high cell vitalization capabilities were observed compared with the water-added control. Thus, it has been confirmed that the samples provide an excellent stress-reducing (anti-stress) action. Alternatively, when the plant extracts (soybean-germ extracts; LGS) described in EXAMPLES 1A and 1C were used, results similar to that in the case where the plant extract described in EXAMPLE 1B was used were also obtained. In summary, by applying polyamines to skin, degradation of fibroblasts due to high-temperature stress is reduced (suppressed) and hence improvement of the feel of skin can be expected.

(EXAMPLE 4A: evaluation of stress-reducing (anti-stress) action of wheat-germ-derived polyamine-containing plant extract (low-temperature stress))

The plant extract (wheat-germ extract; LGW) described in EXAMPLE 2B was used as a plant extract containing wheat-germ-derived polyamine and/or a plant extract containing wheat-germ-derived polyamine as an active component. The evaluation was performed by the following procedure. Fibroblasts (106-05, manufactured by Cell Applications, Inc.) were cultured in a T-75 flask. When the fibroblasts reached 70 to 80% confluency, the culture medium was aspirated. The fibroblasts were washed with 10 ml of PBS twice. The cells were released by the addition of 500 $\mu$l of trypsin/PBS and the reaction was terminated by the addition of 500 $\mu$l of a 10% FCS-containing DMEM (11995-073, manufactured by GIBCO). The cells were mixed with 10 ml of PBS and collected into a 50 ml tube. The cells were mixed with another 10 ml of PBS and collected. The cells were collected at 1,000 rpm for 5 minutes and the supernatant was aspirated. The resultant solution was dissolved in 3 ml of a 1% FCS-containing DMEM and the number of cells was counted. The solution was adjusted to be $4 \times 10^4$ cells/ml ($1 \times 10^4$ cells/250 $\mu$l) with a 1% FCS-containing DMEM and 250 $\mu$l of the resultant solution was each injected into wells of 48-well plates. The cells were incubated overnight in a $CO_2$ incubator set at 37°C. Then, samples (2.5 $\mu$l per well) were added to wells and the cells were incubated for 3 days in a $CO_2$ incubator set at 37°C (under no-stress condition) and set at 32°C (low-temperature stress condition). The experiment was performed with N = 4. After the culture medium was removed with an aspirator, the wells were washed twice with PBS. After 200 $\mu$l of a 5% FCS-containing DMEM (21063-029, manufactured by GIBCO) was added to each well, 20 $\mu$l of a viable-cell count reagent SF (07553-54, manufactured by NACALAI TESQUE, INC.) was added and the reaction was allowed to occur in the $CO_2$ incubator for 5 hours. Abs 490/630 was measured and a cell vitalization percentage was calculated with the following formula.

$$\text{Cell vitalization percentage} = (As - Ab)/(Ac - Ab) \times 100$$

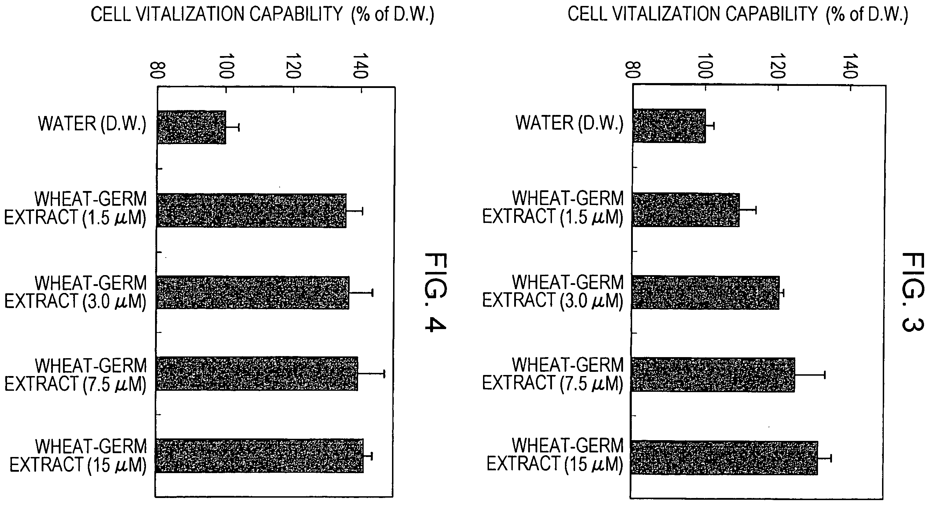

Herein, As represents the Abs 490/630 of a sample; Ac represents the Abs 490/630 of the control (water); and Ab represents the Abs 490/630 of a well to which the cells were not seeded but the 5% FCS-containing DMEM and the viable-cell count reagent SF were added. The evaluation results are illustrated in Fig. 3 in relative values with respect to the cell vitalization action in the control (water added), this cell vitalization action being defined as 100. The concentrations illustrated are the concentrations of polyamine.

The cell vitalization capability of the water-added (control) under the low-temperature stress condition at 32°C was 75% lower than that under the no-stress condition at 37°C (room temperature). As is clear from Fig. 3, in all the culture media to which the wheat-germ extract was added, significantly (significant level: 1% or 5%) high cell vitalization capabilities were observed compared with the water-added control. Thus, it has been confirmed that the samples provide an excellent stress-reducing (anti-stress) action. Alternatively, when the plant extracts (wheat-germ extracts; LGS) described in EXAMPLES 2A and 2C were used, results similar to that in the case where the plant extract described in EXAMPLE 2B was used were also obtained. In summary, by applying polyamines to skin, degradation of fibroblasts due to low-temperature stress is reduced (suppressed) and hence improvement of the feel of skin can be expected.

(EXAMPLE 4B: evaluation of stress-reducing (anti-stress) action of wheat-germ-derived polyamine-containing plant extract (high-temperature stress))

The plant extract (wheat-germ extract; LGW) described in EXAMPLE 2B was used as a plant extract containing wheat-germ-derived polyamine and/or a plant extract containing wheat-germ-derived polyamine as an active component. The evaluation was performed by the following procedure. Fibroblasts (106-05, manufactured by Cell Applications, Inc.) were cultured in a T-75 flask. When the fibroblasts reached 70 to 80% confluency, the culture medium was aspirated. The fibroblasts were washed with 10 ml of PBS twice. The cells were released by the addition of 500 $\mu$l of trypsin/PBS and the reaction was terminated by the addition of 500 $\mu$l of a 10% FCS-containing DMEM (11995-073, manufactured by GIBCO). The cells were mixed with 10 ml of PBS and collected into a 50 ml tube. The cells were mixed with another 10 ml of PBS and collected. The cells were collected at 1,000 rpm for 5 minutes and the supernatant was aspirated. The resultant solution was dissolved in 3 ml of a 1% FCS-containing DMEM and the number of cells was counted. The solution was adjusted to be $4 \times 10^4$ cells/ml ($1 \times 10^4$ cells/250 $\mu$l) with a 1% FCS-containing DMEM and 250 $\mu$l of the resultant solution was each injected into wells of 48-well plates. The cells were incubated overnight in a $CO_2$ incubator set at 37°C. Then, samples (2.5 $\mu$l per well) were added to wells and the cells were incubated for 3 days in a $CO_2$ incubator set at 37°C (under no-stress condition) and set at 42°C (high-temperature stress condition). The experiment was performed with N = 4. After the culture medium was removed with an aspirator, the wells were washed twice with PBS. After 200 $\mu$l of a 5% FCS-containing DMEM (21063-029, manufactured by GIBCO) was added to each well, 20 $\mu$l of a viable-cell count reagent SF (07553-54, manufactured by NACALAI TESQUE, INC.) was added and the reaction was allowed to occur in the $CO_2$ incubator for 5 hours. Abs 490/630 was measured and a cell vitalization percentage was calculated with the following formula.

$$\text{Cell vitalization percentage} = (As - Ab)/(Ac - Ab) \times 100$$

Herein, As represents the Abs 490/630 of a sample; Ac represents the Abs 490/630 of the control (water); and Ab represents the Abs 490/630 of a well to which the cells were not seeded but the 5% FCS-containing DMEM and the viable-cell count reagent SF were added. The evaluation results are illustrated in Fig. 4 in relative values with respect to the cell vitalization action in the control (water added), this cell vitalization action being defined as 100. The concentrations illustrated are the concentrations of polyamine.

The cell vitalization capability of the water-added (control) under the high-temperature stress condition at 42°C was 70% lower than that under the no-stress condition at 37°C (room temperature). As is clear from Fig. 4, in all the culture media to which the wheat-germ extract was added, significantly (significant level: 1% or 5%) high cell vitalization capabilities were observed compared with the water-added control. Thus, it has been confirmed that the samples provide an excellent stress-reducing (anti-stress) action. Alternatively, when the plant extracts (wheat-germ extracts; LGS) described in EXAMPLES 2A and 2C were used, results similar to that in the case where the plant extract described in EXAMPLE 2B was used were also obtained. In summary, by applying polyamines to skin, degradation of fibroblasts due to high-temperature stress is reduced (suppressed) and hence improvement of the feel of skin can be expected.

(EXAMPLE 5A: production of soybean-germ-extract-containing cosmetic lotion)

A cosmetic lotion having the following composition was produced in a standard manner. Another cosmetic lotion not containing any soybean-germ extract was also produced as a control in a standard manner.

| (Composition) | (wt%) |
|---|---|
| Sorbit | 4.0 |
| Dipropylene glycol | 6.0 |
| Polyethylene glycol 1500 | 5.0 |
| POE (20) oleyl alcohol ether | 0.5 |
| Sucrose fatty acid ester | 0.2 |
| Methylcellulose | 0.2 |
| Soybean-germ extract (LGS; polyamine concentration: 3 mM) | 0.5 |
| Purified water | Amount required to make the total amount be 100 |

(EXAMPLE 5B: production of soybean-germ-extract-containing emulsion)

An emulsion having the following composition was produced in a standard manner. Another emulsion not containing any soybean-germ extract was also produced as a control in a standard manner.

| (Composition) | (wt%) |
|---|---|
| Glyceryl ether | 1.5 |
| Sucrose fatty acid ester | 1.5 |
| Sorbitan monostearate | 1.0 |
| Squalane | 7.5 |
| Dipropylene glycol | 5.0 |
| Soybean-germ extract (LGS; polyamine mM) concentration: 3 | 0.5 |
| Purified water | Amount required to make the total amount be 100 |

(EXAMPLE 5C: production of soybean-germ-extract-containing cream)

A cream having the following composition was produced in a standard manner. Another cream not containing any soybean-germ extract was also produced as a control in a standard manner.

| (Composition) | (wt%) |
|---|---|
| Propylene glycol | 6.0 |
| Dibutyl phthalate | 19.0 |
| Stearic acid | 5.0 |
| Glyceryl monostearate | 5.0 |
| Sorbitan monostearate | 12.0 |
| Polyethylene sorbitan monostearate | 38.0 |
| Sodium edetate | 0.03 |
| Soybean-germ extract (LGS; polyamine concentration: 3 mM) | 0.5 |
| Purified water | Amount required to make the total amount be 100 |

(EXAMPLE 6: sensory evaluation of soybean-germ-extract-containing cosmetics (winter: low-temperature stress))

Sensory evaluation was performed with EXAMPLES 5A to 5C. COMPARATIVE EXAMPLES in which no soybean-germ extract was contained were also evaluated. In the sensory evaluation, panels each composed of 20 subjects that were 40 to 60 years old and were suffering from skin symptoms relating to roughness, dryness, glossiness, or the like were asked to use the EXAMPLES and the COMPARATIVE EXAMPLES twice a day for three consecutive months in winter (three months from December to February) during which people are likely to be under low-temperature stress; and the sensory evaluation was performed by using questionnaires asking about the feel of skin (roughness, glossiness, texture, feeling of moistness, feeling of smoothness, and feeling of moistness and elasticity) after the three months. From the results in Table 1, it has been confirmed that the soybean-germ-derived polyamine-containing extract provides an excellent effect of improving the feel of skin compared with the COMPARATIVE EXAMPLES.

[Table 1]

| | | EXAMPLES (with soybean-germ extract) | | | COMPARATIVE EXAMPLES (without soybean-germ extract) | | |
|---|---|---|---|---|---|---|---|
| | | 5A | 5B | 5C | 5A | 5B | 5C |
| Skin-feel improving effect | Enhanced | 15 | 14 | 15 | 0 | 0 | 0 |
| | Somewhat enhanced | 4 | 4 | 3 | 3 | 3 | 2 |
| | No change | 1 | 2 | 2 | 15 | 16 | 16 |
| | Degraded | 0 | 0 | 0 | 2 | 1 | 2 |

(EXAMPLE 7: sensory evaluation of soybean-germ-extract-containing cosmetics (summer: high-temperature stress))

Sensory evaluation was performed with EXAMPLES 5A to 5C. COMPARATIVE EXAMPLES in which no soybean-germ extract was contained were also evaluated. In the sensory evaluation, panels each composed of 20 subjects that were 40 to 60 years old and were suffering from skin symptoms relating to elasticity, greasiness, glossiness, or the like were asked to use the EXAMPLES and the COMPARATIVE EXAMPLES twice a day for three consecutive months in summer (three months from July to September) during which people are likely to be under high-temperature stress; and the sensory evaluation was performed by using questionnaires asking about the feel of skin (greasiness, glossiness, texture, feeling of moistness, feeling of smoothness, and feeling of moistness and elasticity) after the three months. From the results in Table 2, it has been confirmed that the soybean-germ-derived polyamine-containing extract provides an excellent effect of improving the feel of skin compared with the COMPARATIVE EXAMPLES.

[Table 2]

| | | EXAMPLES (with soybean-germ extract) | | | COMPARATIVE EXAMPLES (without soybean-germ extract) | | |
|---|---|---|---|---|---|---|---|
| | - | 5A | 5B | 5C | 5A | 5B | 5C |
| Skin-feel improving effect | Enhanced | 16 | 15 | 16 | 0 | 0 | 0 |
| | Somewhat enhanced | 3 | 4 | 2 | 3 | 3 | 2 |
| | No change | 1 | 1 | 2 | 16 | 17 | 17 |
| | Degraded | 0 | 0 | 0 | 1 | 0 | 1 |

(EXAMPLE 8A: production of wheat-germ-extract-containing cosmetic lotion)

A cosmetic lotion having the following composition was produced in a standard manner. Another cosmetic lotion not containing any wheat-germ extract was also produced as a control in a standard manner.

| (Composition) | (wt%) |
|---|---|
| Sorbit | 4.0 |
| Dipropylene glycol | 6.0 |
| Polyethylene glycol 1500 | 5.0 |
| POE (20) oleyl alcohol ether | 0.5 |
| Sucrose fatty acid ester | 0.2 |
| Methylcellulose | 0.2 |
| Wheat-germ extract (LGW; polyamine concentration: 1.5 mM) | 0.5 |
| Purified water | Amount required to make the total amount be 100 |

(EXAMPLE 8B: production of wheat-germ-extract-containing emulsion)

An emulsion having the following composition was produced in a standard manner. Another emulsion not containing any wheat-germ extract was also produced as a control in a standard manner.

| (Composition) | (wt%) |
|---|---|
| Glyceryl ether | 1.5 |
| Sucrose fatty acid ester | 1.5 |
| Sorbitan monostearate | 1.0 |
| Squalane | 7.5 |
| Dipropylene glycol | 5.0 |
| Wheat-germ extract (LGW; polyamine concentration: 1.5 mM) | 0.5 |
| Purified water | Amount required to make the total amount be 100 |

(EXAMPLE 8C: production of wheat-germ-extract-containing cream)

A cream having the following composition was produced in a standard manner. Another cream not containing any wheat-germ extract was also produced as a control in a standard manner.

| (Composition) | (wt%) |
|---|---|
| Propylene glycol | 6.0 |
| Dibutyl phthalate | 19.0 |
| Stearic acid | 5.0 |
| Glyceryl monostearate | 5.0 |
| Sorbitan monostearate | 12.0 |

(continued)

| (Composition) | (wt%) |
|---|---|
| Polyethylene sorbitan monostearate | 38.0 |
| Sodium edetate | 0.03 |
| Wheat-germ extract (LGW; polyamine concentration: 1.5 mM) | 0.5 |
| Purified water | Amount required to make the total amount be 100 |

(EXAMPLE 9: sensory evaluation of wheat-germ-extract-containing cosmetics (winter: low-temperature stress))

Sensory evaluation was performed with EXAMPLES 8A to 8C. COMPARATIVE EXAMPLES in which no wheat-germ extract was contained were also evaluated. In the sensory evaluation, panels each composed of 20 subjects that were 40 to 60 years old and were suffering from skin symptoms relating to roughness, dryness, glossiness, or the like were asked to use the EXAMPLES and the COMPARATIVE EXAMPLES twice a day for three consecutive months in winter (three months from December to February) during which people are likely to be under low-temperature stress; and the sensory evaluation was performed by using questionnaires asking about the feel of skin (roughness, glossiness, texture, feeling of moistness, feeling of smoothness, and feeling of moistness and elasticity) after the three months. From the results in Table 3, it has been confirmed that the wheat-germ-derived polyamine-containing extract provides an excellent effect of improving the feel of skin compared with COMPARATIVE

EXAMPLES.

[Table 3]

| | | EXAMPLES (with wheat-germ extract) | | | COMPARATIVE EXAMPLES (without wheat-germ extract) | | |
|---|---|---|---|---|---|---|---|
| | | 8A | 8B | 8C | 8A | 8B | 8C |
| Skin-feel improving effect | Enhanced | 17 | 16 | 15 | 0 | 0 | 0 |
| | Somewhat enhanced | 2 | 2 | 3 | 3 | 3 | 2 |
| | No change | 1 | 2 | 2 | 15 | 16 | 16 |
| | Degraded | 0 | 0 | 0 | 2 | 1 | 2 |

(EXAMPLE 10: sensory evaluation of wheat-germ-extract-containing cosmetics (summer: high-temperature stress))

Sensory evaluation was performed with EXAMPLES 8A to 8C. COMPARATIVE EXAMPLES in which no wheat-germ extract was contained were also evaluated. In the sensory evaluation, panels each composed of 20 subjects that were 40 to 60 years old and were suffering from skin symptoms relating to elasticity, greasiness, glossiness, or the like were asked to use the EXAMPLES and the COMPARATIVE EXAMPLES twice a day for three consecutive months in summer (three months from July to September) during which people are likely to be under high-temperature stress; and the sensory evaluation was performed by using questionnaires asking about the feel of skin (greasiness, glossiness, texture, feeling of moistness, feeling of smoothness, and feeling of moistness and elasticity) after the three months. From the results in Table 4, it has been confirmed that the wheat-germ-derived polyamine-containing extract provides an excellent effect of improving the feel of skin compared with the COMPARATIVE EXAMPLES.

[Table 4]

| | | EXAMPLES (with wheat-germ extract) | | | COMPARATIVE EXAMPLES (without wheat-germ extract) | | |
|---|---|---|---|---|---|---|---|
| | | 8A | 8B | 8C | 8A | 8B | 8C |
| Skin-feel improving effect | Enhanced | 18 | 17 | 17 | 0 | 0 | 0 |
| | Somewhat enhanced | 1 | 2 | 2 | 3 | 3 | 2 |
| | No change | 1 | 1 | 1 | 16 | 17 | 17 |
| | Degraded | 0 | 0 | 0 | 1 | 0 | 1 |

(EXAMPLE 11: comparison evaluation in stress-reducing (anti-stress) action (low-temperature stress) among synthetic polyamines, soybean-germ-derived polyamine-containing plant extract, and wheat-germ-derived polyamine-containing plant extract)

As for polyamines, putrescine, spermidine, and spermine were used. The plant extract (soybean-germ extract; LGS) described in EXAMPLE 1B was used as a plant extract containing soybean-germ-derived polyamine. The plant extract (wheat-germ extract; LGW) described in EXAMPLE 2B was used as a plant extract containing wheat-germ-derived polyamine.

The evaluation was performed by the following procedure. Fibroblasts (106-05, manufactured by Cell Applications, Inc.) were cultured in a T-75 flask. When the fibroblasts reached 70 to 80% confluency, the culture medium was aspirated. The fibroblasts were washed with 10 ml of PBS twice. The cells were released by the addition of 500 $\mu$l of trypsin/PBS and the reaction was terminated by the addition of 500 $\mu$l of a 10% FCS-containing DMEM (11995-073, manufactured by GIBCO). The cells were mixed with 10 ml of PBS and collected into a 50 ml tube. The cells were mixed with another 10 ml of PBS and collected. The cells were collected at 1,000 rpm

for 5 minutes and the supernatant was aspirated. The resultant solution was dissolved in 3 ml of a 1% FCS-containing DMEM and the number of cells was counted. The solution was adjusted to be $4 \times 10^4$ cells/ml ($1 \times 10^4$ cells/250 $\mu$l) with a 1% FCS-containing DMEM and 250 $\mu$l of the resultant solution was each injected into wells of 48-well plates. The cells were incubated overnight in a $CO_2$ incubator set at 37°C. Then, samples (2.5 $\mu$l per well) were added to wells and the cells were incubated for 3 days in a $CO_2$ incubator set at 37°C (under no-stress condition) and set at 32°C (low-temperature stress condition). The experiment was performed with N = 4. After the culture medium was removed with an aspirator, the wells were washed twice with PBS. After 200 $\mu$l of a 5% FCS-containing DMEM (21063-029, manufactured by GIBCO) was added to each well, 20 $\mu$l of a viable-cell count reagent SF (07553-54, manufactured by NACALAI TESQUE, INC.) was added and the reaction was allowed to occur in the $CO_2$ incubator for 5 hours. Abs 490/630 was measured and a cell vitalization percentage was calculated with the following formula.

$$\texttt{Cell vitalization percentage = (As - Ab)/(Ac - Ab)} \times 100$$

Herein, As represents the Abs 490/630 of a sample; Ac represents the Abs 490/630 of the control (water); and Ab represents the Abs 490/630 of a well to which the cells were not seeded but the 5% FCS-containing DMEM and the viable-cell count reagent SF were added. The evaluation results are illustrated in Fig. 1 in relative values with respect to the cell vitalization action in a control (water added), this cell vitalization action being defined as 100. The concentrations illustrated are the concentrations of polyamine.

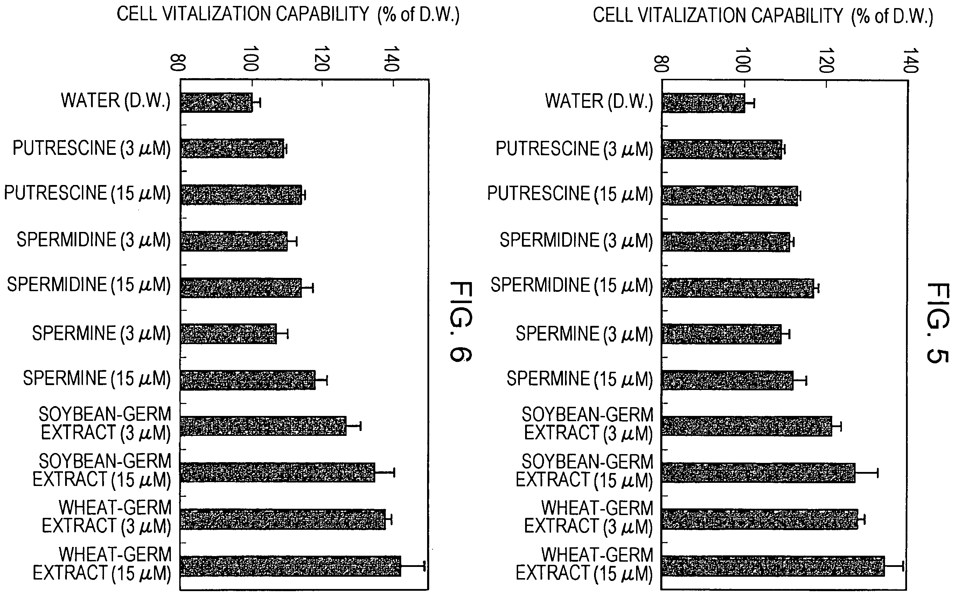

The cell vitalization capability of the water-added (control) under the low-temperature stress condition at 32°C was 75% lower than that under the no-stress condition at 37°C (room temperature). As is clear from Fig. 5, in the culture media to which the soybean-germ-derived polyamine-containing plant extract and the wheat-germ-derived polyamine-containing plant extract were added, significantly (significant level: 1% or 5%) high cell vitalization capabilities were observed compared with the controls to which putrescine, spermidine, and spermine were added. Thus, it has been confirmed that the samples provide an excellent stress-reducing (anti-stress) action. Alternatively, when the plant extracts (soybean-germ extracts; LGS) described in EXAMPLES 1A and 1C and the plant extracts (wheat-germ extracts; LGS) described in EXAMPLES 2A and 2C were used, results similar to those in the cases where the plant extracts described in EXAMPLES 1B and 2B were used were also obtained. In summary, by applying polyamines to skin, degradation of fibroblasts due to low-temperature stress is reduced (suppressed) and hence improvement of the feel of skin can be expected.

(EXAMPLE 12: comparison evaluation in stress-reducing (anti-stress) action (high-temperature stress) among synthetic polyamines, soybean-germ-derived polyamine-containing plant extract, and wheat-germ-derived polyamine-containing plant extract)

As for polyamines, putrescine, spermidine, and spermine were used. The plant extract (soybean-germ extract; LGS) described in EXAMPLE 1B was used as a plant extract containing soybean-germ-derived polyamine. The plant extract (wheat-germ extract; LGW) described in EXAMPLE 2B was used as a plant extract containing wheat-germ-derived polyamine.

The evaluation was performed by the following procedure. Fibroblasts (106-05, manufactured by Cell Applications, Inc.) were cultured in a T-75 flask. When the fibroblasts reached 70 to 80% confluency, the culture medium was aspirated. The fibroblasts were washed with 10 ml of PBS twice. The cells were released by the addition of 500 $\mu$l of trypsin/PBS and the reaction was terminated by the addition of 500 $\mu$l of a 10% FCS-containing DMEM (11995-073, manufactured by GIBCO). The cells were mixed with 10 ml of PBS and collected into a 50 ml tube. The cells were mixed with another 10 ml of PBS and collected. The cells were collected at 1,000 rpm

for 5 minutes and the supernatant was aspirated. The resultant solution was dissolved in 3 ml of a 1% FCS-containing DMEM and the number of cells was counted. The solution was adjusted to be $4 \times 10^4$ cells/ml ($1 \times 10^4$ cells/250 $\mu$l) with a 1% FCS-containing DMEM and 250 $\mu$l of the resultant solution was each injected into wells of 48-well plates. The cells were incubated overnight in a $CO_2$ incubator set at 37°C. Then, samples (2.5 $\mu$l per well) were added to wells and the cells were incubated for 3 days in a $CO_2$ incubator set at 37°C (under no-stress condition) and set at 42°C (high-temperature stress condition). The experiment was performed with N = 4. After the culture medium was removed with an aspirator, the wells were washed twice with PBS. After 200 $\mu$l of a 5% FCS-containing DMEM (21063-029, manufactured by GIBCO) was added to each well, 20 $\mu$l of a viable-cell count reagent SF (07553-54, manufactured by NACALAI TESQUE, INC.) was added and the reaction was allowed to occur in the $CO_2$ incubator for 5 hours. Abs 490/630 was measured and a cell vitalization percentage was calculated with the following formula.

$$\texttt{Cell vitalization percentage = (As - Ab)/(Ac - Ab)} \times$$
$$\texttt{100}$$

Herein, As represents the Abs 490/630 of a sample; Ac represents the Abs 490/630 of the control (water); and Ab represents the Abs 490/630 of a well to which the cells were not seeded but the 5% FCS-containing DMEM and the viable-cell count reagent SF were added. The evaluation results are illustrated in Fig. 2 in relative values with respect to the cell vitalization action in the control (water added), this cell vitalization action being defined as 100. The concentrations illustrated are the concentrations of polyamine.

The cell vitalization capability of the water-added (control) under the high-temperature stress condition at 42°C was 70% lower than that under the no-stress condition at 37°C (room temperature). As is clear from Fig. 6, in the culture media to which the soybean-germ-derived polyamine-containing plant extract and the wheat-germ-derived polyamine-containing plant extract were added, significantly (significant level: 1% or 5%) high cell vitalization capabilities were observed compared with the controls to which putrescine, spermidine, and spermine were added. Thus, it has been confirmed that the samples provide an excellent stress-reducing (anti-stress) action. When the plant extracts (soybean-germ extracts; LGS) described in EXAMPLES 1A and 1C and the plant extracts (wheat-germ extracts; LGS) described in EXAMPLES 2A and 2C were used, results similar to those in the cases where the plant extracts described in EXAMPLES 1B and 2B were used were also obtained. In summary, by applying polyamines to skin, degradation of fibroblasts due to high-temperature stress is reduced (suppressed) and hence improvement of the feel of skin can be expected.

The present invention can provide use of a cosmetic composition that is expected to provide an excellent temperature stress-suppressing action, temperature stress-reducing action and temperature stress-preventing action or the like for cells and the cosmetic composition contains a plant-derived polyamine-containing extract. Furthermore, since the composition can be produced at a lower material cost and on a large scale and is capable of sufficiently withstanding long-term usage, the composition is expected to considerably contribute to the industry.

**Claims**

1.    Use of a cosmetic composition for reducing, suppressing or preventing temperature-stress in skin cells of a mammal, wherein said cosmetic composition comprises, as an active ingredient, a plant-derived polyamine-containing extract, and wherein the temperature stress is due to heat and cold.

2. The use according to claim 1, wherein the plant-derived polyamine-containing extract is an extract derived from at least one material selected from the group consisting of a soybean seed, a soybean germ, a soybean embryo, a soybean bud, a wheat seed, a wheat germ, a wheat embryo, a wheat bud, soymilk, and soybean curd refuse.

3. The use according to claim 1 or 2, wherein the polyamine includes an aliphatic hydrocarbon compound having two or more primary amino groups.

4. The use according to any one of claims 1 to 3, wherein the polyamine is at least one compound selected from the group consisting of 1,3-diaminopropane, putrescine, cadaverine, cardine, spermidine, homospermidine, aminopropylca-daverine, termine, spermine, thermospermine, canavalmine, aminopentylnor-spermidine, N,N-bis(aminopropyl)cadaverine, homospermine, caldopentamine, homocaldopentamine, caldohexamine, and homocaldohexamine.

5. The use according to any one of claims 1 to 4, wherein the polyamine is at least one compound selected from the group consisting of putrescine, spermidine, and spermine.


**Patentansprüche**

1. Verwendung einer kosmetischen Zusammensetzung zum Reduzieren, Unterdrücken oder Verhindern von Temperaturstress in Hautzellen eines Säugetiers, wobei die kosmetische Zusammensetzung als einen Wirkstoff ein von Pflanzen stammendes polyaminhaltiges Extrakt umfasst und wobei der Temperaturstress durch Wärme und Kälte bedingt ist.

2. Verwendung nach Anspruch 1, wobei das von Pflanzen stammende polyaminhaltige Extrakt ein Extrakt ist, das von mindestens einem Material stammt, das aus der Gruppe, bestehend aus einem Sojabohnensamen, einem Sojabohnenkeim, einem Sojabohnenembryo, einer Sojabohnenknospe, einem Weizensamen, einem Weizenkeim, einem Weizenembryo, einer Weizenknospe, Sojamilch und Sojabohnenquarkrückstand, ausgewählt ist.

3. Verwendung nach Anspruch 1 oder 2, wobei das Polyamin eine aliphatische Kohlenwasserstoffverbindung mit zwei oder mehr primären Aminogruppen einschließt.

4. Verwendung nach einem der Ansprüche 1 bis 3, wobei das Polyamin mindestens eine Verbindung, ausgewählt aus der Gruppe, bestehend aus 1,3-Diaminopropan, Putrescin, Cadaverin, Cardin, Spermidin, Homospermidin, Aminopropylcadaverin, Termin, Spermin, Thermospermin, Canavalmin, Aminopentylnorspermidin, N,N-Bis(aminopropyl)cadaverin, Homospermin, Caldopentamin, Homocaldopentamin, Caldohexamin und Homocaldohexamin, ist.

5. Verwendung nach einem der Ansprüche 1 bis 4, wobei das Polyamin mindestens eine Verbindung, ausgewählt aus der Gruppe, bestehend aus Putrescin, Spermidin und Spermin, ist.


**Revendications**

1. Utilisation d'une composition cosmétique pour réduire, supprimer ou empêcher le stress lié à la température dans des cellules cutanées d'un mammifère, dans laquelle ladite composition cosmétique comprend comme ingrédient actif un extrait dérivé de plante contenant de la polyamine, et dans laquelle le stress lié à la température est dû à la chaleur et au froid.

2. Utilisation selon la revendication 1, dans laquelle l'extrait dérivé de plante contenant de la polyamine est un extrait dérivé d'au moins un matériau sélectionné parmi le groupe constitué d'une graine de soja, d'un germe de soja, d'un embryon de soja, d'un bourgeon de soja, d'une graine de blé, d'un germe de blé, d'un embryon de blé, d'un bourgeon de blé, de lait de soja et de déchets de caillé de soja.

3. Utilisation selon la revendication 1 ou 2, dans laquelle la polyamine inclut un composé d'hydrocarbure aliphatique ayant deux groupes amino primaires ou plus.

4. Utilisation selon l'une quelconque des revendications 1 à 3, dans laquelle la polyamine est au moins un composé sélectionné parmi le groupe constitué du 1,3-diaminopropane, de la putrescine, de la cadavérine, de la cardine, de la spermidine, de l'homospermidine, de l'aminopropylcadavérine, de la termine, de la spermine, de la thermospermine, de la canavalmine, de l'aminopentylnorspermidine, de la N,N-bis(aminopropyl)cadavérine, de l'homospermine, de la caldopentamine, de l'homocaldopentamine, de la caldohexamine et de l'homocaldohexamine.

5. Utilisation selon l'une quelconque des revendications 1 à 4, dans laquelle la polyamine est au moins un composé sélectionné parmi le groupe constitué de la putrescine, de la spermidine et de la spermine.

# FIG. 1

# FIG. 2

## FIG. 3

## FIG. 4

24

# FIG. 5

# FIG. 6

## REFERENCES CITED IN THE DESCRIPTION

### Patent documents cited in the description

- JP 2003081868 A
- JP 2001187725 A
- JP 2001213795 A
- JP 2005330213 A
- JP 2005506676 W

### Non-patent literature cited in the description

- *Plant Cell Physiol,* 2002, vol. 43 (2), 196-206
- *J. Nutr. Biochem.,* 1993, vol. 4, 66-70
- *Biosci. Biotech. Biochem.,* 1997, vol. 61 (9), 1582-1584